# EUROPEAN PATENT APPLICATION

(11) **EP 4 059 961 A2**
(43) Date of publication of application: **21.09.2022**
(21) Application number: 22150348.5
(22) Date of filing: 04.02.2016
(51) Int. Cl.: C07K 16/18, C07K 14/705, C07K 14/775, C07K 14/47, C07K 16/46, C12N 15/62, C12N 15/861, A61K 48/00

(54) **ANTI-TAU CONSTRUCTS**

(30) Priority: 04.02.2015 US 201562111924 P; 16.02.2015 US 201562116892 P
(62) Divisional of application: 16706468.2
(71) Applicant: Washington University, Saint Louis, MO 63130 (US)
(72) Inventor: HOLTZMAN, David, St. Louis, 63130 (US); JIANG, Hong, St. Louis, 63130 (US); GALLARDO, Gilbert, St. Louis, 63130 (US); ISING, Christina, 50931 Cologne (DE); LEYNS, Cheryl, St. Louis, 63130 (US)
(74) Representative: J A Kemp LLP

(57) **Abstract**

The present invention provides anti-tau constructs. Anti-tau constructs of the invention are polynucleotide sequences encoding a polypeptide comprising at least one tau binding moiety and optionally comprising a signal peptide and/or a purification moiety. The present invention also provides isolated polypeptides encoded by anti-tau constructs, vectors comprising anti-tau constructs, and isolated cells comprising said vectors.

## Description

### CROSS-REFERENCE TO RELATED APPLICATIONS

This application claims the benefit of priority from U.S. Provisional Application No. 62/111,924, filed on Feb. 4, 2015 and U.S. Provisional Application No. 62/116,892, filed on Feb. 16, 2015, the entire content of each of which is incorporated herein by reference in its entirety.

### FIELD OF THE INVENTION

The present invention provides anti-tau constructs. Anti-tau constructs of the invention are polynucleotide sequences encoding a polypeptide comprising at least one tau binding moiety and optionally comprising a signal peptide and/or a purification moiety. The present invention also provides isolated polypeptides encoded by anti-tau constructs, vectors comprising anti-tau constructs, and isolated cells comprising said vectors.

### BACKGROUND OF THE INVENTION

Anti-tau antibodies and fragments thereof are known in the art. See, for example, PGT/US2013/049333. It has been hypothesized that anti-tau antibodies may be used to target tau for degradation. However, the precise mechanism by which anti-tau antibodies clear tau is unknown. It is also unknown whether the level of degradation is meaningful. Thus, there remains a need in the art to develop molecules capable of effectively targeting tau for degradation.

### REFERENCE TO COLOR FIGURES

The application file contains at least one photograph executed in color. Copies of this patent application publication with color photographs will be provided by the Office upon request and payment of the necessary fee.

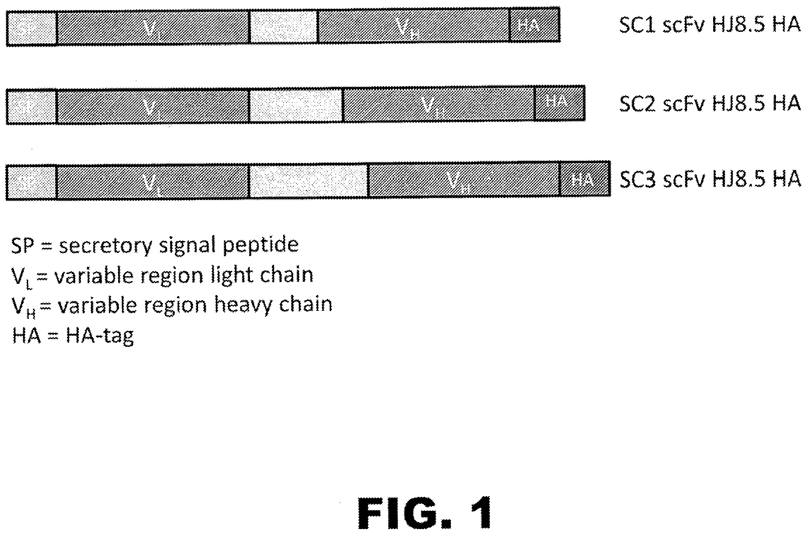

**FIG. 1** depicts a schematic of three examples of single chain variable fragments (scFv) derivatives of HJ8.5. The scFv comprises a secretory signal peptide (SP), a variable region light chain (V_{L}), a spacer (in yellow), a variable region heavy chain (V_{H}), and an HA-tag (HA). SC1 comprises the spacer (GGGS)₁; SC2 comprises the spacer S(GGGS)₂; and SC3 comprises the spacer S(GGGS)₃,

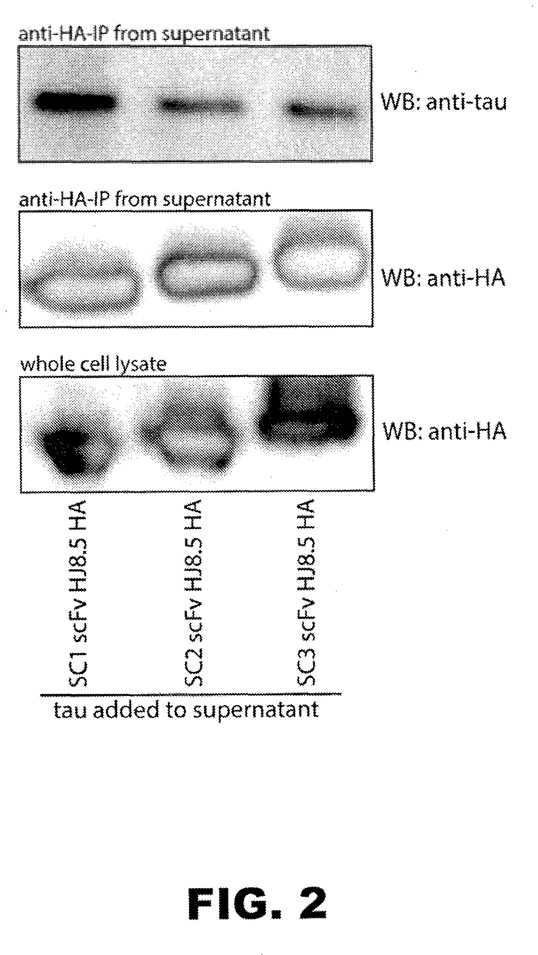

**FIG. 2** depicts Western blots showing that the scFv constructs are expressed and the polypeptides encoded by the constructs are secreted and bind to tau. HEK 293 cells were transfected with the constructs. Both the cell culture supernatant and whole cell lysate were examined for expression of SC1, SC2 or SC3. The top panel shows that SC1, SC2 or SC3 secreted into the supernatant bound to recombinant human tau added to the supernatant. The middle panel shows that the scFv's are secreted into the supernatant. The bottom panel shows that the scFv's are also present in the whole cell lysate.

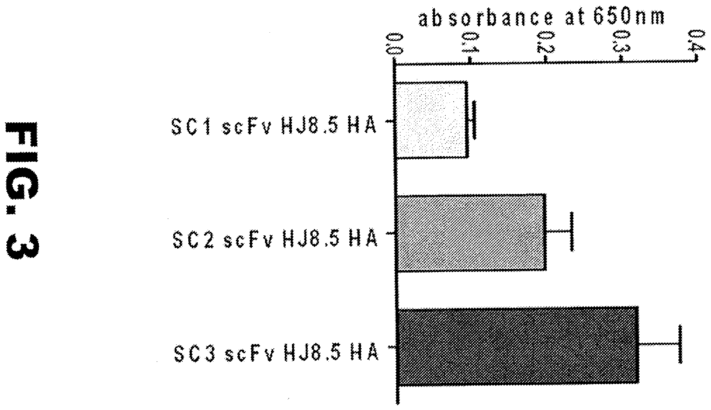

**FIG. 3** depicts a Tau-ELISA showing that the scFv's bind to tau. The constructs were expressed in HEK 293 cells and purified using an anti-HA agarose column. Tau was added at 100 µg/ml onto an ELISA plate and purified scFv's were then added. An anti-HA antibody coupled to HRP was used for detection. As evidenced by the ELISA, the three scFv's all bound to tau.

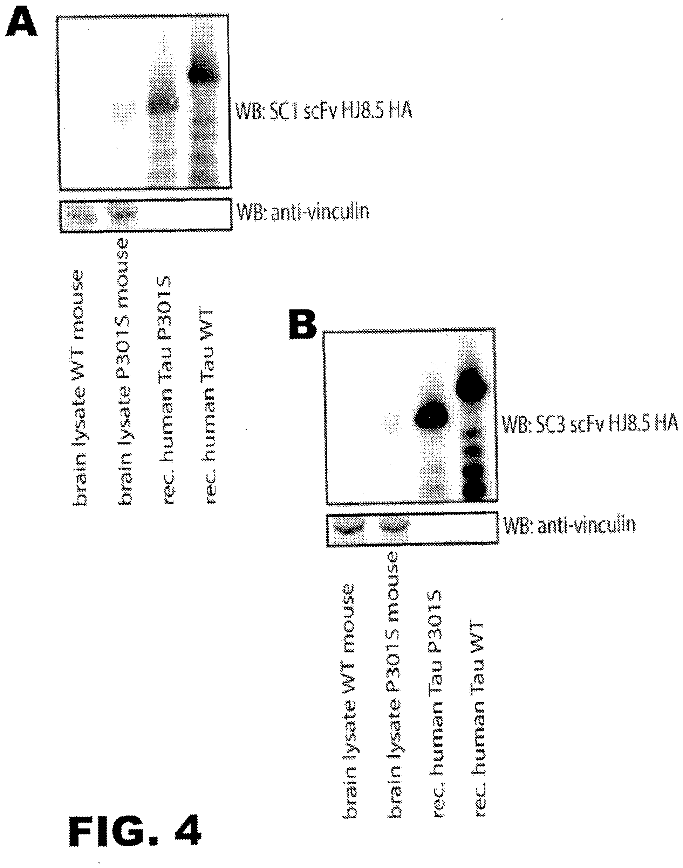

**FIG. 4** depicts Western blots showing that the scFv's detect human but not mouse tau. Mouse brain lysate comprising tau and recombinant human tau were both run on a gel and the scFv's SC1 (**A**) and SC3 (**B**) were used to detect tau. Recombinant human tau was detected by SC1 and SC3; mouse tau was not. Anti-vinculin Western blot was used as a loading control for tissue samples.

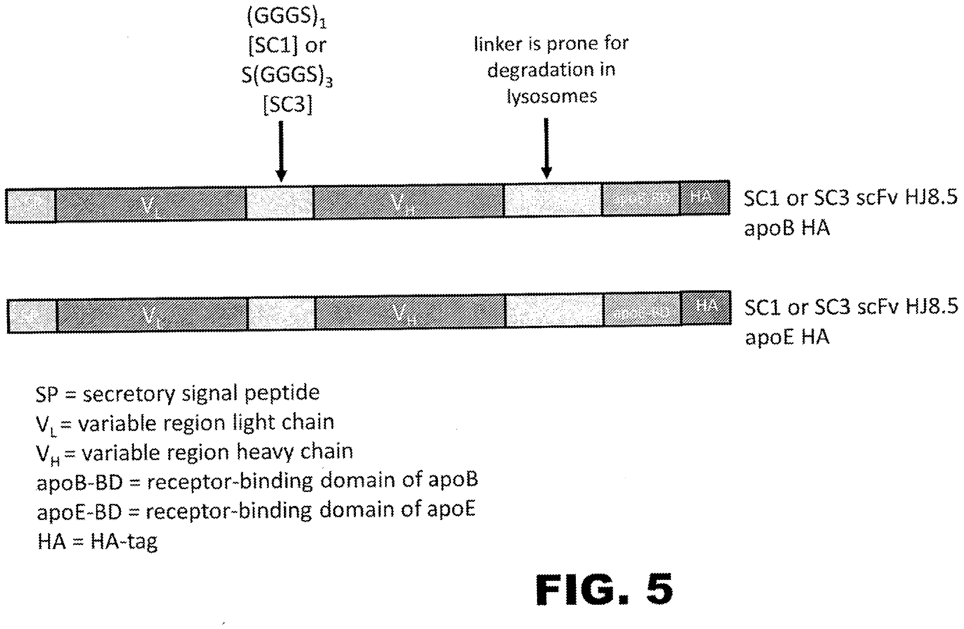

**FIG. 5** depicts a schematic of single chain variable fragments (scFv) derived from HJ8.5 further comprising receptor binding domains of apoB/apoE. The anti-tau construct comprises a secretory signal peptide (SP), a variable region light chain (V_{L}), a spacer (in yellow), a variable region heavy chain (V_{H}), a linker (in yellow), a receptor binding domain of ApoB or ApoE (ApoB-BD or ApoE-BD) and an HA-tag (HA). SC1 comprises the spacer (GGGS)₁; and SC3 comprises the spacer S(GGGS)₃. The linker comprises the sequence S(GGGS)₄. The receptor binding domain of ApoB and/or ApoE may be used to target the polypeptide encoded by the construct for LDLR/LRP1-mediated cellular clearance.

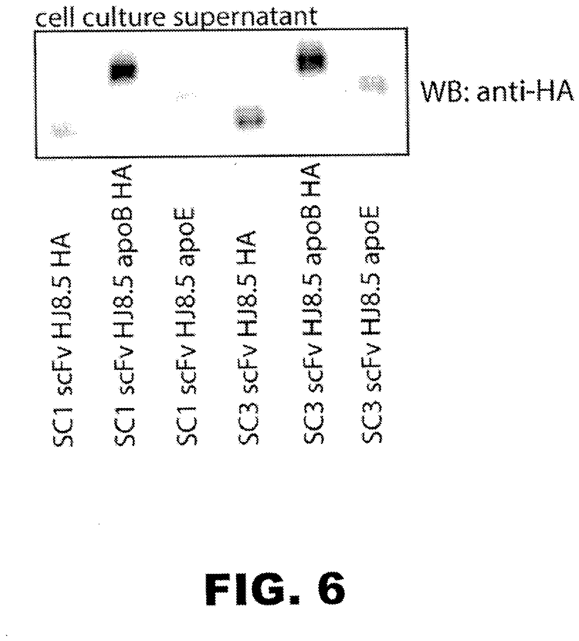

**FIG. 6** depicts a Western blot showing that the polypeptides encoded by the anti-tau constructs comprising the receptor binding domains of apoB/apoE are secreted. Using an HA antibody, polypeptides encoded by all 6 constructs were detected in the cell culture supernatant. SC1 and SC1 comprising the receptor binding domains of ApoB or ApoE and SC3 and SC3 comprising the receptor binding domains of ApoB or ApoE.

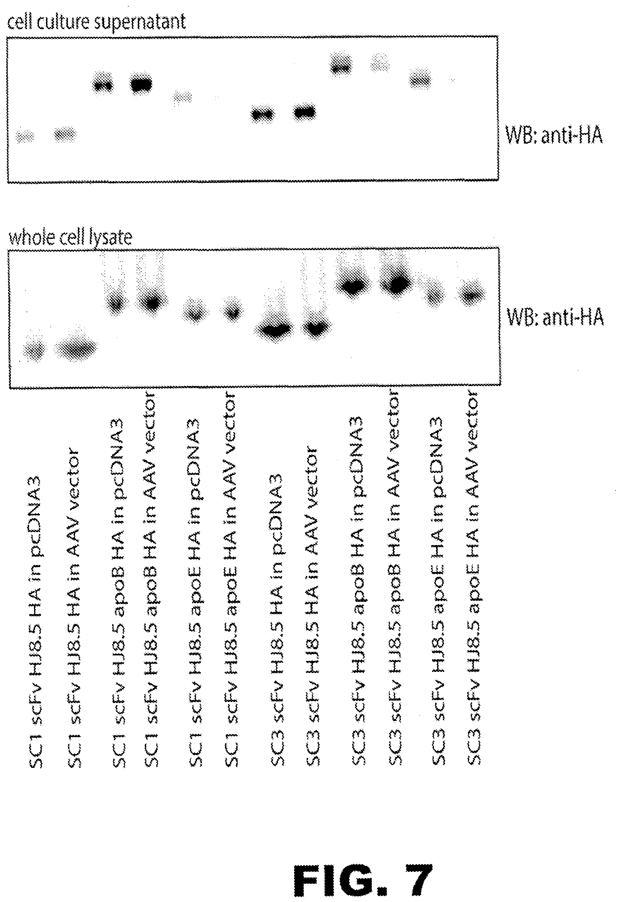

**FIG. 7** depicts a Western blot showing that anti-tau constructs may also be expressed and polypeptides encoded by the anti-tau constructs secreted using an AAV vector. The top panel shows that all 6 anti-tau constructs are expressed and polypeptides encoded by the constructs secreted into the supernatant using either the pcDNA3 plasmid or an AAV vector. The bottom panel shows that all 6 anti-constructs are expressed and polypeptides encoded by the constructs are present in the whole cell lysate using either the pcDNA3 plasmid or an AAV vector.

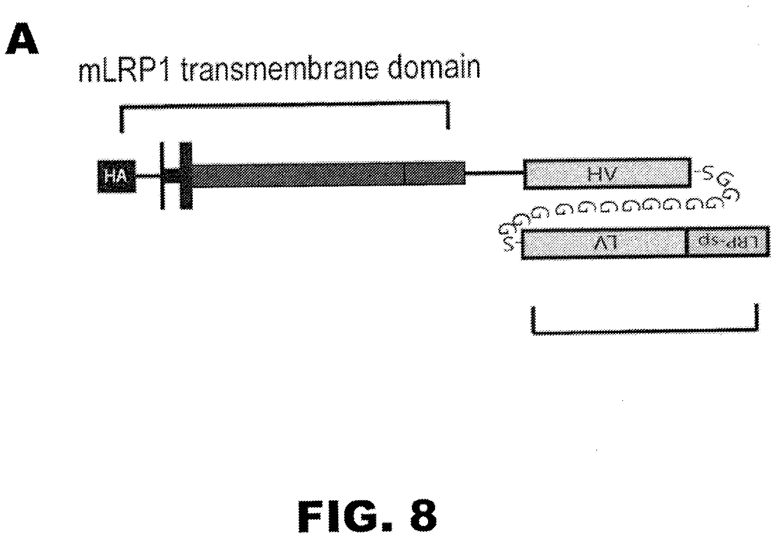

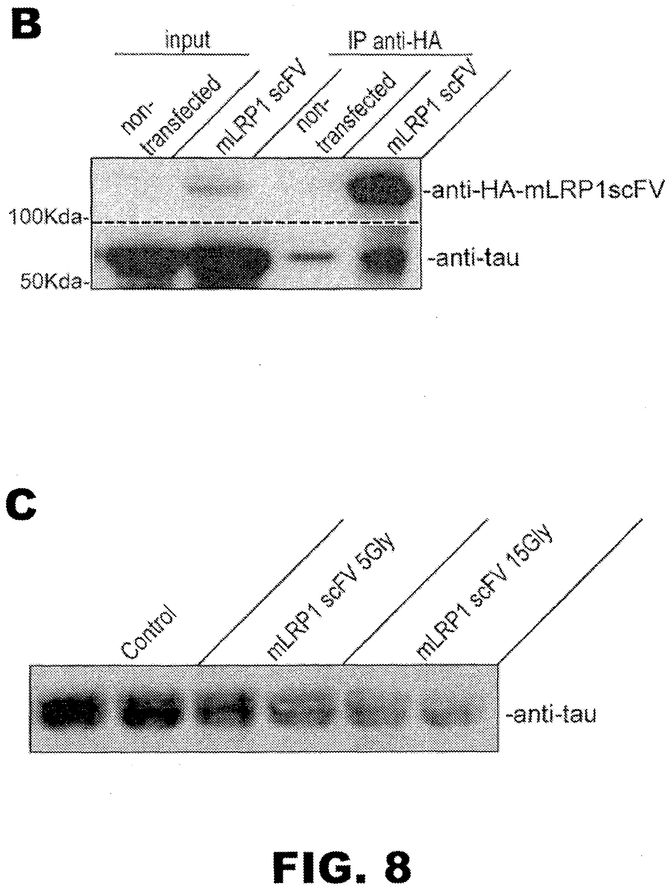

**FlG. 8** depicts a schematic and Western blots showing expression of an scFv derived from HJ8.5 fused to the transmembrane and intracellular domain of LRP1. (**A**) Blue depicts the LRP1 transmembrane domain, orange depicts the variable region, the letters connecting the variable domains indicate the linker, and the green depicts the HA tag. (**B**) depicts a Western blot showing that the LRP1 scFv construct is expressed and the polypeptide encoded therefrom binds tau using anti-HA immunoprecipitation (IP). (**C**) depicts an anti-tau Western blot showing that the polypeptide encoded by the LRP1 scFv construct binds tau in the media, leading to cellular uptake and degradation, as well as less tau in the media. Two constructs were tested, one with 5Gly and one with 15Gly.

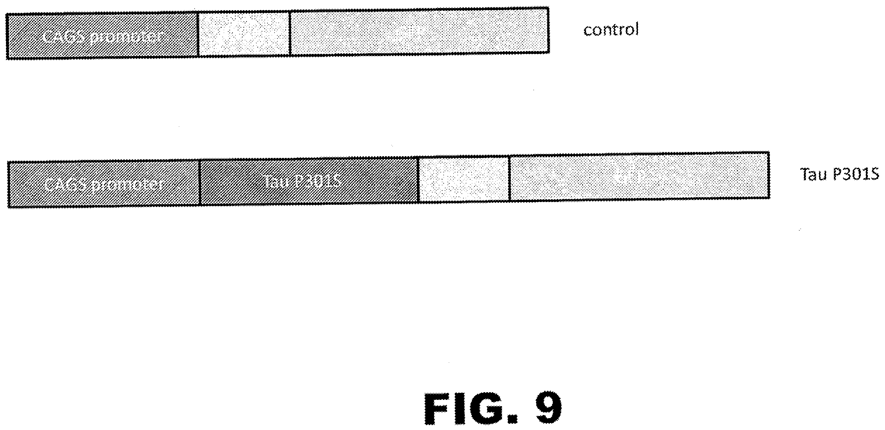

**FIG. 9** depicts a schematic of AAV constructs used to express proteins in the brain. Tau P301S AAV2/8 construct and control AAV2/8 construct were constructed as depicted. The control construct comprises CAGS promoter, IRE and GFP and the Tau P301S construct comprises CAGS promoter, Tau, IRE and GFP. The constructs may be used to express proteins in brain for rapid assessment,

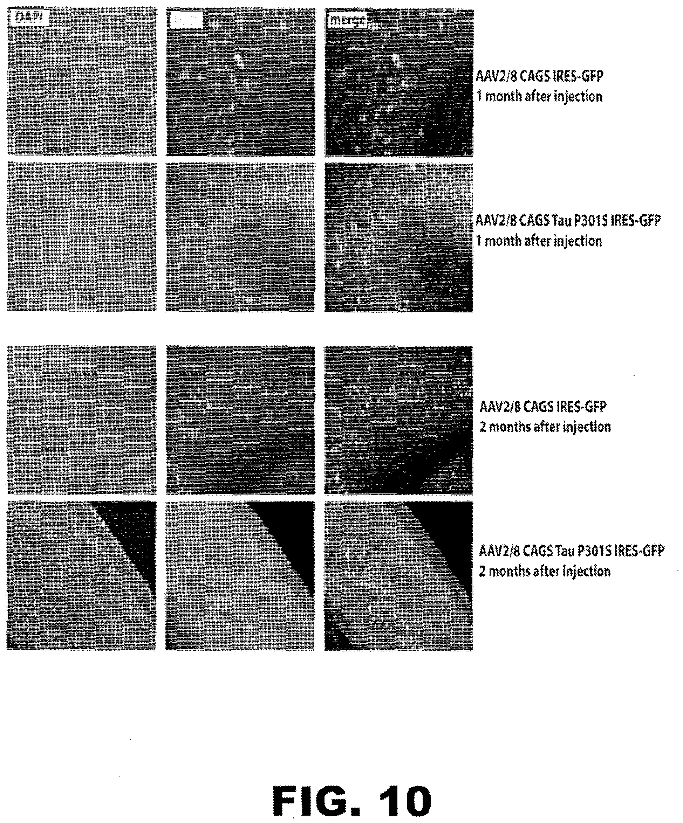

**FIG. 10** depicts images showing the expression of the constructs in mice. AAV vectors expressing tau resulted in high expression of GFP at both 1 month and 2 months after injection.

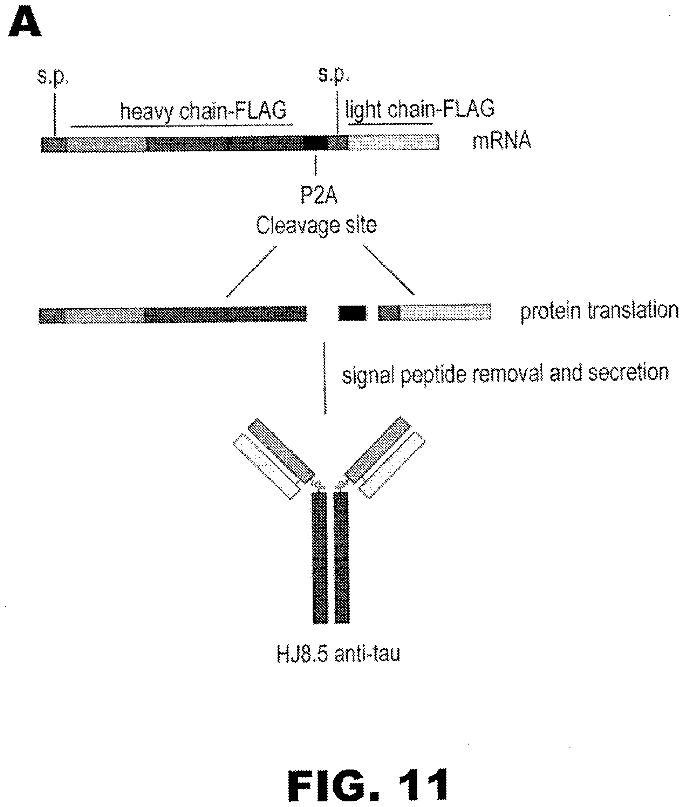

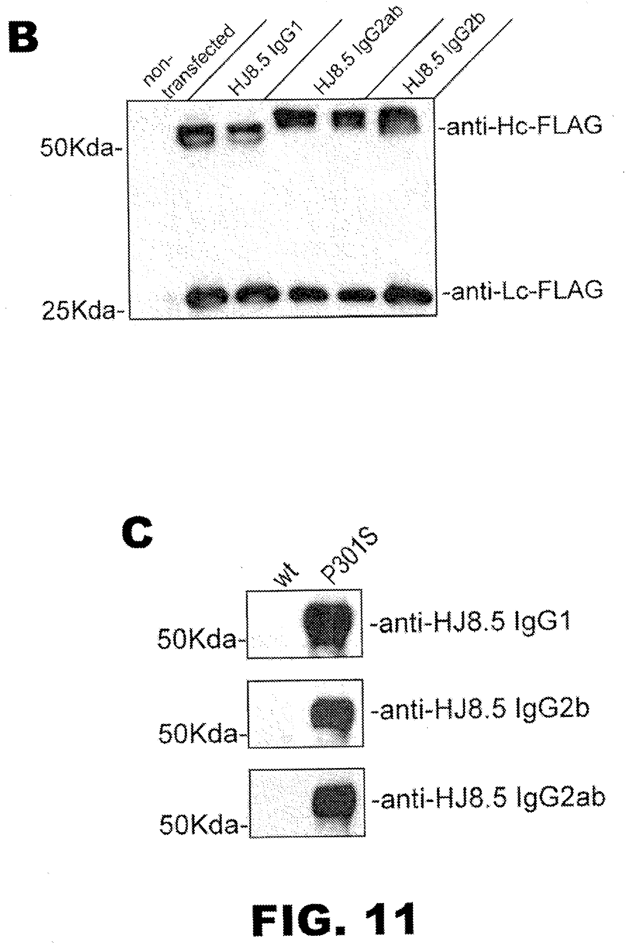

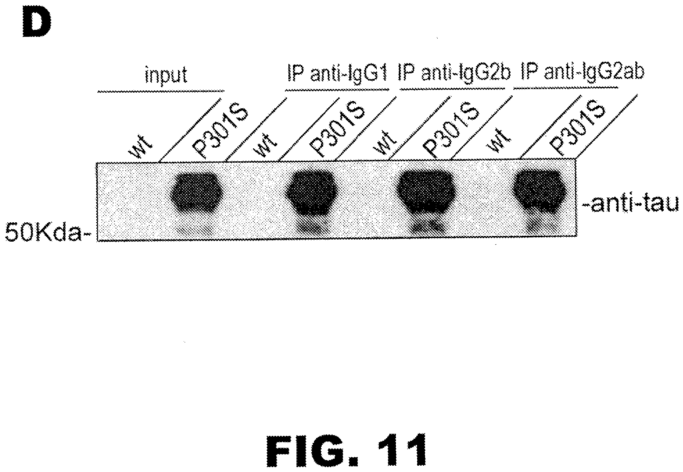

**FIG. 11** depicts a schematic and Western blots of the characterization of the anti-tau antibody HJ8.5 with different Fc domains. (**A**) depicts a schematic of the constructs to construct anti-tau antibody HJ8.5. (**B**) depicts a Western blot showing that the HJ8.5 antibody constructs comprising IgG1, IgG2ab and IgG2b are expressed upon transfection. (**C**) depicts a Western blot showing that the three constructs of HJ8.5 antibody comprising IgG1, IgG2ab and IgG2b bind tau P301S. (**D**) depicts a Western blot showing that immunoprecipitated HJ8.5 antibody comprising IgG1, IgG2ab and IgG2b pull down tau.

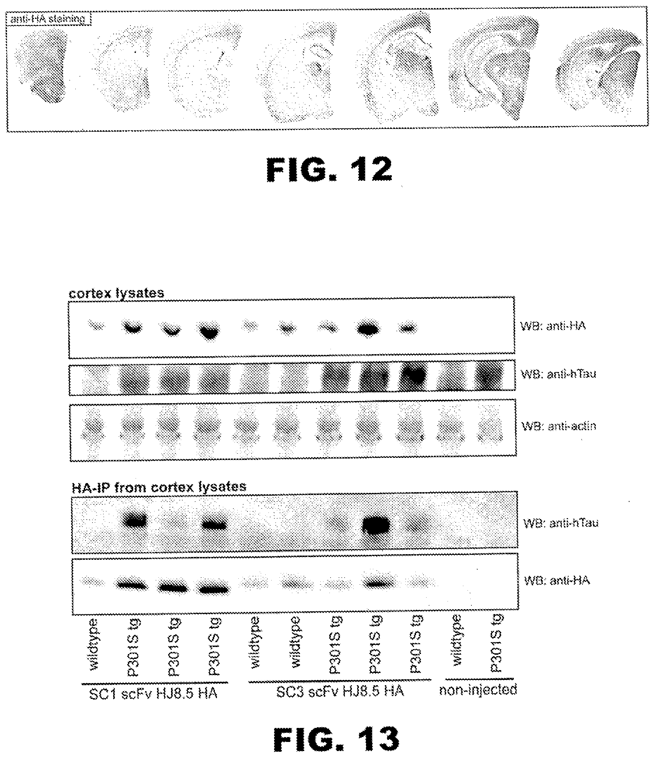

**Fig. 12** depicts an anti-HA staining of brain sections from a mouse that was injected with SC3 scFv HJ8.5 HA AAV2/8 into the ventricles at post-natal day (P) 0 and euthanized 3 months later. The scFvs show good expression throughout the brain.
**Fig. 13** depicts a Western Blot of cortex lysates (top panel) from different mice injected with either SC1 scFv HJ8.5 HA AAV2/8 or SC3 scFv HJ8.5 HA AAV2/8 into the ventricles at P0, All injected mice show expression of the scFvs. Immunoprecipitation experiments (bottom panel) with an HA antibody showed coprecipitation of human tau from mice that expressed the human P301S tau transgene, but not wild type mice (bottom panel).

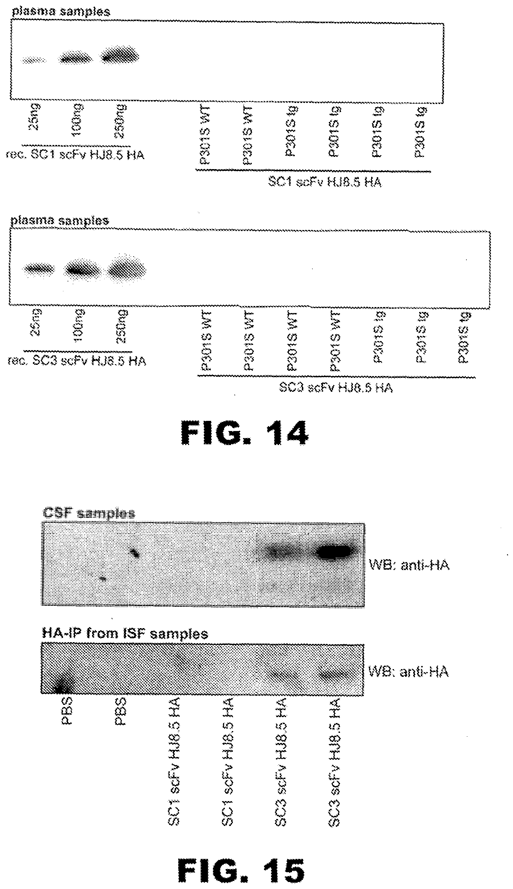

**Fig. 14** depicts a Western Blot of plasma samples of mice injected with SC1 scFv HJ8.5 HA AAV2/8 (top panel) or SC3 scFv HJ8.5 HA AAV2/8 (bottom panel) into the ventricles at P0. The Blot was stained with an HA antibody, showing that scFvs are not detectable in the plasma.
**Fig. 15** depicts a Western Blot of CSF samples (top panel) of mice injected with SC1 scFv HJ8.5 HA AAV2/8 (top panel), SC3 scFv HJ8.5 HA AAV2/8 or PBS into the ventricles at P0. In these samples, only SC3 scFv HJ8.5 HA could be detected. An immunoprecipitation experiment with a HA antibody revealed the presence of SC3 scFv HJ8.5 HA but not SC1 scFv HJ8.5 HA in ISF samples from these mice.

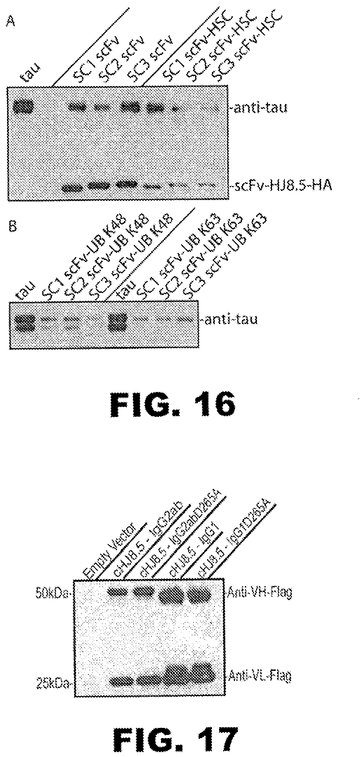

**Fig. 16** depicts a Western Blot of scFv HJ8.5 intrabodies C-terminally HA tagged that target tau. (A) 293t cells transfected with tau and co-transfected with SC1, SC2, SC3 intrabodies or intrabodies fused to HSC-binding motifs for chaperone-mediated autophagy. (B) 293t cells transfected with tau and co-transfected with SC1, SC2, SC3 intrabodies or intrabodies fused to either ubiquitin K48R point mutation for lysosomal degradation, ubiquitin K63R point mutation for proteasomal degradation.
**Fig. 17** depicts a Western blot showing that the cHJ8.5 antibody constructs comprising IgG2ab, IgG2abD265A, IgG1, and IgG1D265A that are C-terminally Flag tagged at the heavy and light IgG chains are expressed and secreted upon transfection in CHOK1 cells.
**Fig, 18** depicts Western blots showing characterization of AAV-cHJ8.5 IgG Fc variants in primary cultures. A depicts expression of full-length chimeric cHJ8.5 IgG2ab, IgG2abD265A, IgG1, and IgG1D265A constructs following infection with respective AAV2/8-cHJ8.5 viruses in primary neurons and glia cultures. B depicts the supernatant from the primary cultures can be used to detect human tau run on a Western blot in brain lysate from P301S human tau transgenic mice. IgG variants are C-terminally Flag tagged at the heavy and light IgG chains.

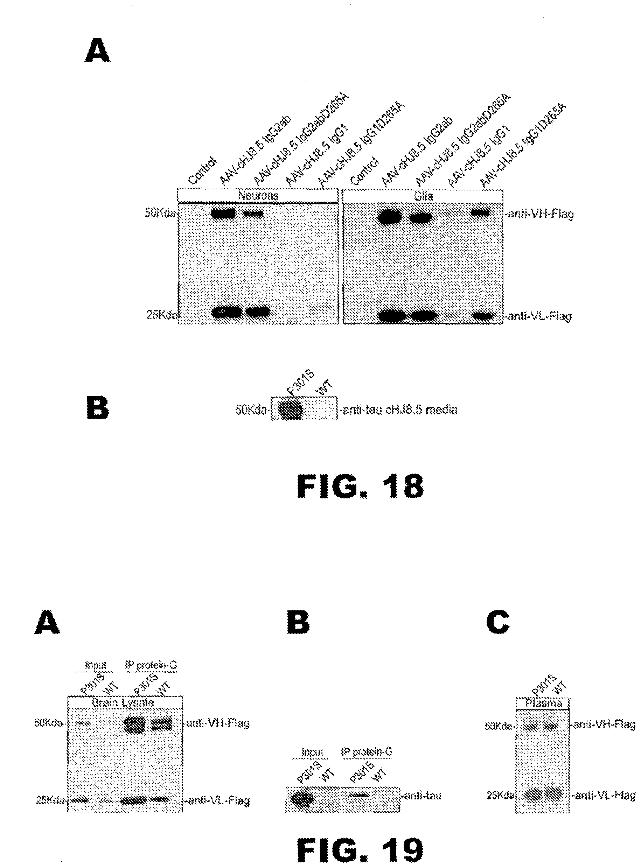

**Fig. 19** depicts Western blots characterizing expression and secretion of AAV2/8-cHJ8.5 IgG2ab in vivo. A depicts a Western blot of cortical brain lysate from a P301S human tau transgenic mice and their WT littermate injected at postnatal day 0 (P0) with AAV-cHJ8.5 IgG2ab-Flag. Protein G effectively immunoprecipitates anti-tau cHJ8.5 heavy and light chains from P301S and control brain lysates. B shows that protein G co-immunoprecipitates cHJ8.5 and human tau in P301S brain lysates. C depicts cHJ8.5-Flag is secreted and detected in the plasma of mice injected with AAV2/8 cHJ8.5.

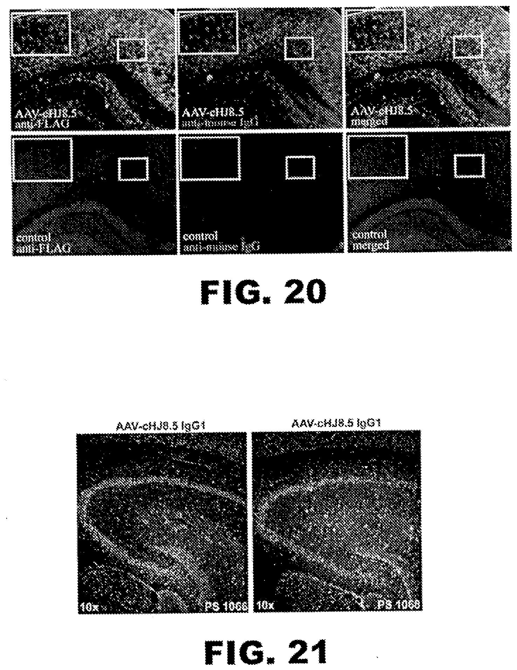

**Fig. 20** depicts immunohistochemistry demonstrating wide-spread expression of AAV-cHJ8.5 IgG2ab-Flag (upper panel) versus AAV-control (lower panel) treated P301S brain sections at 5 weeks post injection as indicated by probing for either anti-flag (green) or anti-IgG mouse (red).
**Fig. 21** depicts immunohistochemistry demonstrating wide-spread expression of AAV-cHJ8.5 IgG1-Flag treated brain sections at 12 weeks post injection as indicated by probing for anti-flag.

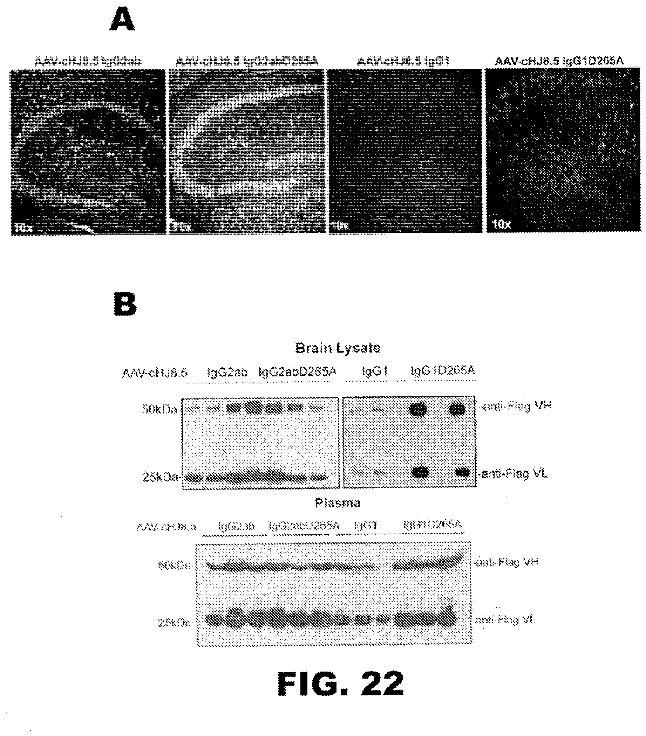

**Fig. 22** depicts expression of all cHJ8.5 IgG Fc variants C termianlly Flag tagged in vivo following injection with AAV2/8-cHJ8.5-Flag viruses at P0. A depicts immunohistochemistry of AAV-cHJ8.5 IgG Fc variant treated mouse brain sections at 14 days post-injection as indicated by probing with anti-Flag. B depicts expression of cHJ8.5 variants in mice 14 days post-injection by Western blot in cortex brain lysate (upper panel) and in plasma (lower panel).

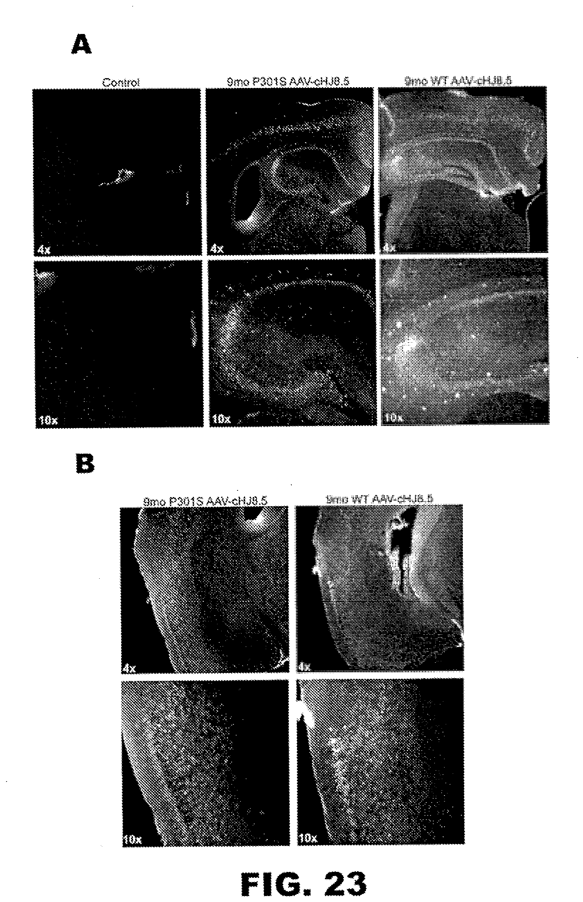

**Fig. 23** depicts immunohistochemistry demonstrating wide-spread expression of AAV218-cHJ8.5 IgG2ab-Flag treated brain sections at 9 months post-injection as indicated by probing with anti-IgG mouse. (A) depicts expression in the hippocampus of 9 month old mice while (B) depicts expression in the entorhinal cortex of 9 month old mice expressing cHJ8.5 IgG2ab.

### DETAILED DESCRIPTION

A "targeting moiety" refers to a polypeptide that is able to direct the entity to which it is attached (e.g., a tau binding moiety) to a target site. Target sites may include, but are not limited to, the cell surface, a cell-surface protein, and an intracellular vesicle. In one embodiment, a targeting moiety may comprise a binding domain derived from a target receptor ligand. A target receptor ligand is a ligand that binds a target receptor. Suitable target receptors include cell-surface receptors capable of receptor-mediated endocytosis and lysosomal targeting. Non-limiting examples of suitable target receptors include the low-density lipoprotein receptor (LDLR), the low-density lipoprotein receptor-related protein (LRP1), transferrin receptors, and mannose-6-phosphate receptor. LDLR and LRP1 are cell-surface receptors that recognize the apolipoproteins ApoE and ApoB. Non-limiting examples of target receptor ligands for LDLR and LRP1 are Apolipoprotein E (ApoE) and Apolipoprotein B (ApoB). Amino acid sequences comprising the binding domains of ApoE and ApoB are provided herein. In another embodiment, a targeting moiety may comprise an antibody capable of specifically binding to an antigenic determinant on a target site, or a fragment thereof that retains specific binding to the antigenic determinant. The human transferrin receptor is a cell-surface receptor that recognizes transferrin and human hemochromatosis (HFE) protein. For a review of targeted drug delivery via the transferrin receptor-mediated endocytosis pathway, see Qian ZM et al. Pharmacological Reviews (2002) 54(4): 561-587. In yet another embodiment, a targeting moiety may comprise an aptamer capable of specifically binding to an antigenic determinant on a target site. In another embodiment, a targeting moiety may comprise a transmembrane domain and/or intracellular domain of a cell-surface protein. In certain embodiments, the cell-surface protein is a cell-surface receptor capable of receptor-mediated endocytosis. Non-limiting examples of suitable cell-surface receptors include the low-density lipoprotein receptor (LDLR), the low-density lipoprotein receptor-related protein (LRP1), transferrin receptors, and mannose-6-phosphate receptor. Amino acid sequences comprising the transmembrane and intracellular domains of LRP1 are provided herein. Other non-limiting examples of targeting moiety are binding motifs of a Heat Shock Cognate protein (HSC), such as Hsc70, that mediate chaperone-mediated autophagy; and ubiquitin or ubiquitin mutant, such as ubiquitin having a K48R point mutation for lysosomal degradation or a K63R point mutation for proteasomal degradation.

A "tau binding moiety" refers to a polypeptide that specifically binds to an antigenic determinant of tau. The tau binding moiety can be any type of antibody or fragment thereof that retains specific binding to an antigenic determinant of tau. Antibody fragments include, but are not limited to, V_{H} fragments, V_{L} fragments, Fab fragments, F(ab')₂ fragments, scFv fragments, Fv fragments, minibodies, diabodies, triabodies, and tetrabodies (see, e.g., Hudson and Souriau, Nature Med. 9: 129-134 (2003)). "Specific binding" is meant that the binding is selective for the tau antigen and can be discriminated from unwanted or non-speciffic interactions. In one embodiment, the anti-tau constant comprises at least one, at least two, at least three or more antigen binding moieties.

An "antigenic determinant of tau" is synonymous with "tau antigen" and "tau epitope," and refers to a site (e.g., a contiguous stretch of amino acids or a conformational configuration made up of different regions of non-contiguous amino acids) on tau (linear or folded) to which a tau binding moiety binds, forming a tau binding moiety-antigen complex.

The term "antibody" is used in the broadest sense and specifically covers, for example, single monoclonal antibodies (including agonist, antagonist, and neutralizing antibodies), antibody compositions with polyepitopic specificity, polyclonal antibodies, single chain anti-antibodies, and fragments of antibodies (see below) as long as they specifically bind a native polypeptide and/or exhibit a biological activity or immunological activity of this invention. "Monoclonal antibody" refers to an antibody that is derived from a single copy or clone, including e.g., any eukaryotic, prokaryotic, or phage clone. "Monoclonal antibody" is not limited to antibodies produced through hybridoma technology. Monoclonal antibodies can be produced using e.g., hybridoma techniques well known in the art, as well as recombinant technologies, phage display technologies, synthetic technologies or combinations of such technologies and other technologies readily known in the art. Furthermore, the monoclonal antibody may be labeled with a detectable label, immobilized on a solid phase and/or conjugated with a heterologous compound (e.g., an enzyme or toxin) according to methods known in the art.

"Antibody fragments" comprise a portion of an intact antibody, preferably the antigen binding or variable region of the intact antibody, In some contexts herein, fragments will be mentioned specifically for emphasis; nevertheless, it will be understood that regardless of whether fragments are specified, the term "antibody" includes such fragments as well as single-chain forms. As long as the polypeptide retains an ability to specifically bind its intended target, it is Included within the term "antibody." Examples of antibody fragments include Fab, Fab', F(ab')2, and Fv fragments; V_{H} fragments, V_{L} fragments; single chain variable fragments (scFv); diabodies; triabodies; tetrabodies; linear antibodies; single-chain antibody molecules; and multispecific antibodies formed from antibody fragments. See, for example, Hudson and Souriau, Nature Med. 9: 129-134 (2003); and Holliger et al., Proc. Natl. Acad. Sci. USA 90: 644-6448 (1993).

The expression "linear antibodies" generally refers to the antibodies described in Zapata et al., Protein Eng., 8(10):1057-1062 (1995), and U.S. Pat. No. 5,641,870, Example 2. Briefly, these antibodies comprise a pair of tandem Fd segments (V_{H}-C_{H1}-V_{H}-C_{H1} which, together with complementary light chain polypeptides, form a pair of antigen binding regions. Linear antibodies can be bispecific or monospecific.

"Fv" is the minimum antibody fragment which contains a complete antigen-recognition and -binding site. This fragment consists of a dimer of one heavy- and one light-chain variable region domain in tight, non-covalent association. From the folding of these two domains emanate six hypervariable loops (3 loops each from the H and L chain) that contribute the ammo acid residues for antigen binding and confer antigen binding specificity to the antibody. However, even a single variable domain (or half of an Fv comprising only three CDRs specific for an antigen) has the ability to recognize and bind antigen, although at a lower affinity than the entire binding site.

A "single-chain Fv" comprises the V_{H} and V_{L} antibody domains connected into a single polypeptide chain. Preferably, the sFv polypeptide further comprises a polypeptide linker between the V_{H} and V_{L} domain which enables the sFv to form the desired structure for antigen binding. The length of the polypeptide linker can and vary. In some embodiments, the polypeptide linker is at least 2, 3, 4, 5, 6, 7, 8, 9, 10, 11, 12, 13, 14, 15, 16, 17,18, 19, 20 or more amino acids in length. A single-chain Fv can be abbreviated as "sFv" or "scFv." For a review of sFv, see Pluckthun in The Pharmacology of Monoclonal Antibodies, vol. 113, Rosenburg and Moore eds., Springer-Verlag, New York, pp. 269-315 (1994); Borrebaeck 1995, infra.

The term "diabodies" refers to small antibody fragments prepared by constructing sFv fragments (see preceding paragraph) with short linkers (about 1-10 residues, preferably about 1-5 residues or about 5-10 residues, or even about 1-3 residues or about 3-5 residues) between the V_{H} and V_{L} domains such that inter-chain but not intra-chain pairing of the V domains is achieved, resulting in a bivalent fragment, i.e., fragment having two antigen-binding sites. Bispecific diabodies are heterodimers of two "crossover" sFv fragments in which the V_{H} and V_{L} domains of the two antibodies are present on different polypeptide chains. Diabodies are described more fully in, for example, EP 404,097; WO 93/11161; and Hollinger et al., PNAS USA, 90:6444-6448 (1993).

A "spacer" refers to the part of an scFv linking the V_{H} fragment and the V_{L} fragment. In most instances, but not all, the spacer is a peptide. A spacer peptide may be from about 1 to about 50 amino acids in length, preferably about 4 to about 25 amino acids in length, or about 4 to about 15 amino acids in length. Typically, spacer peptides with less than about 4 or about 5 amino acids in length do not allow variable regions to fold together. A spacer peptide may be comprised of any suitable combination of amino acids that provides sufficient flexibility and solubility. Preferably, a linker peptide is rich in glycine, as well as serine or threonine. In an exemplary embodiment, a spacer comprises the amino acid sequence (GGGS/T)ₙ or S/T(GGGS/T)ₙ, wherein *n* is an integer from 1 to 10, inclusive,

A "linker" refers to the moiety attaching a tau binding moiety and a targeting moiety. In most instances, but not all, the linker is a peptide. In certain embodiments, the linker may be prone for degradation in lysosomes. A linker peptide may be from about 1 to about 50 amino acids in length, preferably about 4 to about 25 amino acids in length, or about 4 to about 15 amino acids in length. A linker peptide may be comprised of any suitable combination of amino acids that provides sufficient flexibility and solubility. Preferably, a linker peptide is rich en glycine, as well as serine or threonine, In an exemplary embodiment, a spacer comprises the amino acid sequence (GGGS/T)ₙ or S/T(GGGS/T)ₙ, wherein *n* is an integer from 1 to 6, inclusive.

The term "polynucleotide" is intended to encompass a singular nucleic acid as well as plural nucleic acids, and refers to an isolated nucleic acid molecule or construct, e.g., messenger RNA (mRNA), cDNA, or vector DNA. A polynucleotide may comprise a conventional phosphodiester bond or a nonconventional bond (e.g., an amide bond, such as found in peptide nucleic acids (PNA)). The term "nucleic acid" refers to any one or more nucleic acid segments, e.g., DNA or RNA fragments, present in a polynucleotide. By "isolated" nucleic acid or polynucleotide is intended a nucleic acid molecule, DNA or RNA, which has been removed from its native environment. For example, a recombinant polynucleotide encoding a tau binding moiety contained in a vector is considered isolated for the purposes of the present invention. Further examples of an isolated polynucleotide include recombinant polynucleotides maintained in heterologous host cells or purified (partially or substantially) polynucleotides in solution. Isolated RNA molecules include *in vivo* or *in vitro* RNA transcripts of polynucleotides of the present invention. Isolated polynucleotides or nucleic acids according to the present invention further include such molecules produced synthetically. In addition, polynucleotide or a nucleic acid may be or may include a regulatory element such as a promoter, ribosome binding site, or a transcription terminator.

As used herein, a "coding region" is a portion of nucleic acid which consists of codons translated into ammo acids. Although a "stop codon" (TAG, TGA, or TAA) is not translated into an amino acid, it may be considered to be part of a coding region, but any flanking sequences, for example promoters, ribosome binding sites, transcriptional terminators, introns, and the like, are not part of a coding region. Two or more coding regions of the present invention can be present in a single polynucleotide construct, e.g., on a single vector, or in separate polynucleotide constructs, e.g., on separate (different) vectors. Furthermore, any vector may contain a single coding region, or may comprise two or more coding regions, e.g., a single vector may separately encode an immunoglobulin heavy chain variable region and an immunoglobulin light chain variable region. In addition, a vector, polynucleotide, or nucleic acid of the invention may encode heterologous coding regions, either fused or unfused to a nucleic acid encoding a binding molecule, an antibody, or fragment, variant, or derivative thereof. Heterologous coding regions include without limitation specialized elements or motifs, such as a signal peptide or a heterologous functional domain.

In certain embodiments, the polynucleotide or nucleic acid is DNA. in the case of DNA, a polynucleotide comprising a nucleic acid which encodes a polypeptide normally may include a promoter and/or other transcription or translation control elements operably associated with one or more coding regions. An operable association is when a coding region for a gene product, e.g., a polypeptide, is associated with one or more regulatory sequences in such a way as to place expression of the gene product under the influence or control of the regulatory sequence(s). Two DNA fragments (such as a polypeptide coding region and a promoter associated therewith) are "operably associated" or "operably linked" if induction of promoter function results in the transcription of mRNA encoding the desired gene product and if the nature of the linkage between the two DNA fragments does not interfere with the ability of the expression regulatory sequences to direct the expression of the gene product or interfere with the ability of the DNA template to be transcribed. Thus, a promoter region would be operably associated with a nucleic acid encoding a polypeptide if the promoter was capable of effecting transcription of that nucleic acid. The promoter may be a cell-specific promoter that directs substantial transcription of the DNA only in predetermined cells. Other transcription control elements, besides a promoter, for example enhancers, operators, repressors, and transcription termination signals, can be operably associated with the polynucleotide to direct cell-specific transcription. Suitable promoters and other transcription control regions are disclosed herein.

A variety of transcription control regions are known to those skilled in the art The term "control regions" refers to DNA sequences necessary for the expression of an operably linked coding sequence in a particular host organism. The control regions that are suitable for prokaryotes, for example, include a promoter, optionally an operator sequence, and a ribosome binding site. Eukaryotic cells are known to utilize promoters, polyadenylation signals, and enhancers. These include, without limitation, transcription control regions which function in vertebrate cells, such as, but not limited to, promoter and enhancer segments from cytomegaloviruses (the immediate early promoter, in conjunction with intron-A), simian virus 40 (the early promoter), and retroviruses (such as Rous sarcoma virus). Other transcription control regions include those derived from vertebrate genes such as actin, heat shock protein, bovine growth hormone and rabbit β-globin, as well as other sequences capable of controlling gene expression in eukaryotic cells. Additional suitable transcription control regions include tissue-specific promoters and enhancers as well as lymphokine-inducible promoters (e.g., promoters inducible by interferons or interleukins),

Similarly, a variety of translation control elements are known to those of ordinary skill in the art. These include, but are not limited to ribosome binding sites, translation initiation and termination codons, and elements derived from picornaviruses (particularly an internal ribosome entry site, or IRES, also referred to as a CITE sequence).

In other embodiments, a polynucleotide of the present invention is RNA, for example, in the form of messenger RNA (mRNA).

Polynucleotide and nucleic acid coding regions of the present invention may be associated with additional coding regions which encode secretory or signal peptides, which direct the secretion of a polypeptide encoded by a polynucleotide of the present invention. According to the signal hypothesis, proteins secreted by mammalian cells have a signal peptide or secretory leader sequence which is cleaved from the mature protein once export of the growing protein chain across the rough endoplasmic reticulum has been initiated. Those of ordinary skill in the art are aware that polypeptides secreted by vertebrate cells generally have a signal peptide fused to the N-terminus of the polypeptide, which is cleaved from the complete or "full-length" polypeptide to produce a secreted or "mature" form of the polypeptide. In certain embodiments, the native signal peptide, e.g., an immunoglobulin heavy chain or light chain signal peptide is used, or a functional derivative of that sequence that retains the ability to direct the secretion of the polypeptide that is operably associated with it. Alternatively, a heterologous mammalian signal peptide, or a functional derivative thereof, may be used. For example, the wild-type leader sequence may be substituted with the leader sequence of human tissue plasminogen activator (TPA) or mouse β-glucuronidase,

A "polypeptide" is intended to encompass a singular "polypeptide" as well as plural "polypeptides," and refers to a molecule composed of monomers (amino acids) linearly linked by amide bonds (also known as peptide bonds). The term "polypeptide" refers to any chain or chains of two or more amino acids, and does not refer to a specific length of the product. Thus, peptides, dipeptides, tripeptides, oligopeptides, "protein," "amino acid chain," or any other term used to refer to a chain or chains of two or more amino acids, are included within the definition of "polypeptide," and the term "polypeptide" may be used instead of, or interchangeably with any of these terms.

The term "polypeptide" is also intended to refer to the products of post-expression modifications of the polypeptide, including without limitation glycosylation, acetylation, phosphorylation, amidation, derivatization by known protecting/blocking groups, proteolytic cleavage, or modification by non-naturally occurring amino acids. A polypeptide may be derived from a natural biological source or produced by recombinant technology, but is not necessarily translated from a designated nucleic acid sequence. It may be generated in any manner, including by chemical synthesis.

A polypeptide of the invention may be of a size of about 3 or more, 5 or more, 10 or more, 20 or more, 25 or more, 50 or more, 75 or more, 100 or more, 200 or more, 500 or more, 1,000 or more, or 2,000 or more amino acids. Polypeptides may have a defined three-dimensional structure, although they do not necessarily have such structure. Polypeptides with a defined three-dimensional structure are referred to as folded, and polypeptides which do not possess a defined three-dimensional structure, but rather can adopt a large number of different conformations, and are referred to as unfolded. As used herein, the term glycoprotein refers to a protein coupled to at least one carbohydrate moiety that is attached to the protein via an oxygen-containing or a nitrogen-containing side chain of an amino acid residue, e.g., a serine residue or an asparagine residue.

"Isolated," when used to describe the various polypeptides disclosed herein, means a polypeptide has been identified and separated and/or recovered from a cell or cell culture from which it was expressed. No particular level of purification is required. For example, an isolated polypeptide can be removed from its native or natural environment. Recombinantly produced polypeptides and proteins expressed in host cells are considered isolated for purposed of the invention, as are native or recombinant polypeptides which have been separated, fractionated, or partially or substantially purified by any suitable technique.

An "isolated" nucleic acid encoding a polypeptide or other polypeptide-encoding nucleic acid is a nucleic acid molecule that is identified and separated from at least one contaminant nucleic acid molecule with which it is ordinarily associated in the natural source of the polypeptide-encoding nucleic acid. An isolated polypeptide-encoding nucleic acid molecule is other than in the form or setting in which it is found in nature. Isolated polypeptide-encoding nucleic acid molecules therefore are distinguished from the specific polypeptide-encoding nucleic acid molecule as it exists in natural cells. However, an isolated polypeptide-encoding nucleic acid molecule includes polypeptide-encoding nucleic acid molecules contained in cells that ordinarily express the polypeptide where, for example, the nucleic acid molecule is in a chromosomal location different from that of natural cells

An "isolated" cell is a cell isolated from a native source.

A "signal peptide" or "signal sequence" is a short peptide present at the N-terminus of a newly synthesized polypeptide that targets the polypeptide towards the secretory pathway. Generally a signal peptide is about 5 to about 30 amino acids in length, and has a common structure that may comprise a positively charged n-region, followed by a hydrophobic h-region and a neutral but polar c-region. Signal peptide databases provide access to single peptide sequences found in mammals, Drosophila, viruses, bacteria, and yeast. The choice of the signal peptide can and will vary depending on a variety factors including, but not limited to, the desired cellular location and type of cell. In an exemplary embodiment, a signal peptide may be SEQ ID NO: 21 (MDMRVPAQLLGLLLLWLRGARC), or SEQ ID NO: 23 (MLTPPLLLLLPLLSALVAAAIDAP).

A "purification moiety" is intended to encompass any molecule that facilitates the purification of a polynucleotide or, more preferably, a polypeptide of the invention including, but not limited to, biotin, avidin, stretpavidin, protein A, protein G, antibodies or fragments thereof, polyhistidine, Ni2+, Flag tags, myc tags. In preferred embodiments, a purification moiety comprises a peptide tag useful for purification include, but are not limited to, the "HA" tag, which corresponds to an epitope derived from the influenza hemagglutinin protein (Wilson et al., Cell 37 (1984), 767) and the "flag" tag. Purification moieties may further comprise a cleavage site to remove the moiety.

A "subject" includes, but is not limited to, a human, a livestock animal, a companion animal, a lab animal, and a zoological animal. In one embodiment, the subject may be a rodent, e.g. a mouse, a rat, a guinea pig, etc. In another embodiment, the subject may be a livestock animal. Non-limiting examples of suitable livestock animals may include pigs, cows, horses, goats, sheep, llamas and alpacas. In yet another embodiment, the subject may be a companion animal. Non-limiting examples of companion animals may include pets such as dogs, cats, rabbits, and birds. In yet another embodiment, the subject may be a zoological animal. As used herein, a "zoological animal" refers to an animal that may be found in a zoo. Such animals may include non-human primates, large cats, wolves, and bears. In preferred embodiments, the animal is a laboratory animal. Non-limiting examples of a laboratory animal may include rodents, canines, felines, and non-human primates. In certain embodiments, the animal is a rodent. Non-limiting examples of rodents may include mice, rats, guinea pigs, etc. In embodiments where the animal is a mouse, the mouse may be a C57BL/6 mouse, a Balb/c mouse, a 129sv, or any other laboratory strain. In an exemplary embodiment, the subject is a C57BL/6J mouse. In a preferred embodiment, the subject is human.

The term "tau" is intended to encompass all types and forms of the tau protein including, but not limited to, tau monomers, tau aggregates, tau fibrils, tau seeds, tau oligomers, as well as all phosphorylated forms of tau.

In humans, there are six isoforms of tau that are generated by alternative splicing of exons 2, 3, and 10. The isoforms ranging from 352 to 441 amino acids. Exons 2 and 3 encode 29-amino acid inserts each in the N-terminus (called N, and hence, tau isoforms may be 2N (both inserts), 1N (exon 2 only), or 0N (neither). All tau isoforms have three repeats of the microtubule binding domain. Inclusion of exon 10 at the C-terminus leads to inclusion of a fourth microtubule binding domain encoded by exon 10. Hence, tau isoforms may be comprised of four repeats of the microtubule binding domain (exon 10 included) or three repeats of the microtubule binding domain (exon 10 excluded). The amino acid sequence of tau can be retrieved from the literature and pertinent databases; see Goedert et al., Proc. Natl. Acad. Sci. USA 85 (1988), 4051-4055, Goedert et al., EMBO J. 8 (1989), 393-399, Goedert et al., EMBO J. 9 (1990), 4225-4230 and GenBank UniProtKB/swissprot: locus TAU_HUMAN, accession numbers P10636-2 (Fetal-tau) and P10636-4 to -8 (Isoforms B to F).

Another striking feature of tau protein is phosphorylation, which occurs at about 30 of 79 potential serine (Ser) and threonine (Thr) phosphorylation sites. Tau is highly phosphorylated during the brain development. The degree of phosphorylation declines in adulthood. Some of the phosphorylation sites are located within the microtubule binding domains of tau, and it has been shown that an increase of tau phosphorylation negatively regulates the binding of microtubules.

### I Tau binding moiety

In an aspect, the present invention provides a tau binding moiety. A "tau binding moiety" refers to a polypeptide that specifically binds to an antigenic determinant of tau. The tau binding moiety can be any type of antibody or fragment thereof that retains specific binding to an antigenic determinant of tau. Antibody fragments that retain specific binding to an antigenic determinant include, but are not limited to, Fab fragments, F(ab')₂ fragments, scFv fragments, Fv fragments, V_{H} fragments, V_{L} fragments, minibodies, diabodies, triabodies, and tetrabodies (see, e.g., Hudson and Souriau, Nature Med. 9: 129-134 (2003)). In some embodiments, when a tau binding moiety is encoded by a polynucleotide, the tau binding moiety is selected from the group consisting of scFv fragments, V_{H} fragments, V_{L} fragments, minibodies, diabodies, triabodies, tetrabodies, and linear antibodies. Suitable tau binding moieties are known in the art. For example, see PCT/US2013/049333, hereby incorporated by reference in its entirety.

In some embodiments, a tau binding moiety is an anti-tau antibody. The term "antibody" is defined above, and hereby incorporated in this section by reference. In all instances, an anti-tau antibody of the invention specifically binds tau. In exemplary embodiments, an antibody of the invention specifically binds human tau. Generally, anti-tau antibodies bind to tau with an affinity constant or affinity of interaction (KD) in the range of 0,1 pM to 10 nM, with a preferred range being 0.1 pM to 1 nM. The sequence of tau from a variety of species is known in the art, and methods of determining whether an antibody binds to tau are known in the art.

The basic antibody unit of an antibody useful herein comprises a tetramer. Each tetramer is composed of two identical pairs of polypeptide chains, each pair having one "light' (about 25 kDa) and one "heavy" chain (about 50-70 kDa). The amino-terminal portion of each chain includes a variable region of about 100 to 110 or more amino acids primarily responsible for antigen recognition. The carboxy-terminal portion of each chain defines a constant region primarily responsible for effector function.

Light chains are classified as gamma, mu, alpha, and lambda. Heavy chains are classified as gamma, mu, alpha, delta, or epsilon, and define the antibody's isotype as IgO, IgM, IgA, IgD and IgE, respectively. Within light and heavy chains, the variable and constant regions are joined by a "J" region of about 12 or more amino acids, with the heavy chain also including a "D" region of about 10 more amino acids.

The variable regions of each light/heavy chain pair form the antibody binding site. Thus, an intact antibody has two binding sites. The chains exhibit the same general structure of relatively conserved framework regions (FR) joined by three hypervariable regions, also called complementarily determining regions (hereinafter referred to as "CDRs.") The CDRs from the two chains are aligned by the framework regions, enabling binding to a specific epitope. From N-terminal to C-terminal, both light and heavy chains comprise the domains FR1, CDR1, FR2, CDR2, FR3, CDR3 and FR4 respectively. The assignment of amino acids to each domain is in accordance with known conventions (See, Kabat "Sequences of Proteins of Immunological Interest" National Institutes of Health, Bethesda, Md., 1987 and 1991; Chothia, et al, J. Mol. Bio. (1987) 196:901-917; Chothia, et al., Nature (1989) 342:878-883).

In some embodiments, a tau binding moiety is an anti-tau monoclonal antibody. Monoclonal anti-tau antibodies are generated with appropriate specificity by standard techniques of immunization of mammals, forming hybridomas from the antibody-producing cells of said mammals or otherwise immortalizing them, and culturing the hybridomas or immortalized cells to assess them for the appropriate specificity, In the present case, such antibodies could be generated by immunizing a human, rabbit, rat or mouse, for example, with a peptide representing an epitope encompassing a region of the tau protein coding sequence or an appropriate subregion thereof. Materials for recombinant manipulation can be obtained by retrieving the nucleotide sequences encoding the desired antibody from the hybridoma or other cell that produces it. These nucleotide sequences can then be manipulated and isolated, characterized, purified and, recovered to provide them in humanized form, for use herein if desired. Included within the definition of monoclonal antibody is "humanized antibody."

In some embodiments, a tau binding moiety is a humanized anti-tau antibody. As used herein "humanized anti-tau antibody" includes an anti-tau antibody that is composed partially or fully of amino acid sequences derived from a human antibody germline by altering the sequence of an antibody having non-human complementarity determining regions ("CDR"). The simplest such alteration may consist simply of substituting the constant region of a human antibody for the murine constant region, thus resulting in a human/murine chimera which may have sufficiently low immunogenicity to be acceptable for pharmaceutical use. Preferably, however, the variable region of the antibody and even the CDR is also humanized by techniques that are by now well known in the art. The framework regions of the variable regions are substituted by the corresponding: human framework regions leaving the non-human CDR substantially intact, or even replacing the CDR with sequences derived from a human genome. CDRs may also be randomly mutated such that binding activity and affinity for tau is maintained or enhanced in the context of fully human germline framework regions or framework regions that are substantially human. Substantially human frameworks have at least 90%, 95%, or 99% sequence identity with a known human framework sequence. Fully useful human antibodies may also be produced in genetically modified mice whose immune systems have been altered to correspond to human immune systems. As mentioned above, it is sufficient for use in the methods of this discovery, to employ an immunologically specific fragment of the antibody, including fragments representing single chain forms.

Further, as used herein the term "humanized antibody" refers to an anti-tau antibody comprising a human framework, at least one CDR from a nonhuman antibody, and in which any constant region present is substantially identical to a human immunoglobulin constant region, i.e., at least about 85-90%, preferably at least 95% identical. Hence, all parts of a humanized antibody, except possibly the CDRs, are substantially identical to corresponding pairs of one or more native human immunoglobulin sequences.

If desired, the design of humanized immunoglobulins may be carried out as follows. When an amino acid falls under the following category, the framework amino acid of a human immunoglobulin to be used (acceptor immunoglobulin) is replaced by a framework amino acid from a CDR-providing nonhuman immunoglobulin (donor immunoglobulin): (a) the amino acid in the human framework region of the acceptor immunoglobulin is unusual for human immunoglobulin at that position, whereas the corresponding amino acid in the donor immunoglobulin is typical for human immunoglobulin at that position; (b) the position of the amino acid is immediately adjacent to one of the CDRs; or (c) any side chain atom of a framework amino acid is within about 5-6 angstroms (center-to-center) of any atom of a CDR amino acid in a three dimensional immunoglobulin model (Queen, et al., op. cit., and Co, et al, Proc. Natl. Acad. Sci. USA (1991) 88:2869). When each of the amino acids in the human framework region of the acceptor immunoglobulin and a corresponding amino acid in the donor immunoglobulin is unusual for human immunoglobulin at that position, such an amino acid is replaced by an amino acid typical for human immunoglobulin at that position. Other methods are known in the art and are also suitable. See, for example, Jones TD et al. Methods Molecular Biology (2009): 525: 405-423.

In some embodiments, a tau binding moiety is a single chain variable fragment (scFv). A scFv is comprised of the heavy and light chain variable regions connected by a spacer. In most instances, but not all, the spacer may be a peptide, A spacer peptide may be from about 1 to about 50 amino acids in length, preferably about 4 to about 25 amino acids in length, or about 4 to about 15 amino acids in length. Typically spacer peptides with less than about 4 or about 5 amino acids in length do not allow variable regions to fold together. A spacer peptide may be comprised of any suitable combination of amino acids that provides sufficient flexibility and solubility. Preferably, a linker peptide is rich in glycine, as well as serine or threonine. Methods of making and using scFv's are known in the art. In some embodiments, a spacer peptide of an scFv comprises a polypeptide sequence that is (GGGS/T)ₙ or S/T(GGGS/T)ₙ. In other embodiments, a spacer peptide of an scFv consists essentially of a polypeptide sequence that is (GGGS/T)ₙ or S/T(GGGS/T)ₙ. In preferred embodiments, the scFv's of the present invention are attached to a targeting moiety via a linker that is the same or different than the spacer connecting the heavy and light chain variable regions.

In some embodiments, a tau binding moiety is a diabody. The "diabody" technology described by Hollinger et al., PNAS USA, 90:6444-6448 (1993) has provided an alternative mechanism for making bispecific antibody fragments, The fragments comprise a V_{H} connected to a V_{L} by a spacer which is too short to allow pairing between the two domains on the same chain. Accordingly, the V_{H} and V_{L} domains of one fragment are forced to pair with the complementary V_{L} and V_{H} domains of another fragment, thereby forming two antigen-binding sites. Another strategy for making bispecific antibody fragments by the use of single-chain Fv (sFv) dimers has also been reported. See Gruber et al., J. Immunol., 152:5368 (1994).

### (a) Exemplary embodiments

A tau binding moiety of the present invention preferably recognizes one of several epitopes. In one embodiment, a tau binding moiety of the present invention recognizes a tau epitope with the amino acid sequences listed in **Table A**, and is selected from the group consisting of a monoclonal antibody, a Fab fragment, a F(ab')₂ fragment, a V_{H} fragment, a V_{L} fragment, a Fv fragment, a single-chain variable fragment (scFv), a minibody, a diabody, a triabody, and a tetrabody. In preferred embodiments, the tau binding moiety is an scFv.

**Table A. Tau epitopes**

| Antibody Name | Tau epitope |
|---|---|
| HJ8.1.1 | DRKDQGGYTMHQD (SEQ ID NO: 1) |
| HJ8.1.2 | TDHGAE (SEQ ID NO: 10) |
| HJ8.2 | PRHLSNV (SEQ ID NO: 3) |
| HJ8.3 | PRHLSNV (SEQ ID NO:3) |
| HJ8.4 | KTDHGA (SEQ ID NO: 11) |
| HJ8.5 | DRKDQGGYTMHQD (SEQ ID NO: 1) |
| HJ8.7 | AAGHV (SEQ ID NO: 5) |
| HJ8.8 | EPRQ (SEQ ID NO: 4) |
| HJ9.1 | TDHGAEIVYKSPVVSG (SEQ ID NO: 6) |
| HJ9.2 | EFEVMED (SEQ ID NO: 7) |
| HJ9.3 | GGKVQIINKK (SEQ ID NO: 8) |
| HJ9.4 | EFEVMED (SEQ ID NO: 7) |
| HJ9.5 | EFEVMED (SEQ ID NO: 7) |

In another embodiment, a tau binding moiety of the present invention that specifically binds to tau recognizes an epitope within the amino acid sequences of SEQ ID NO: 1 (DRKDQGGYTMHQD), and is selected from the group consisting of a monoclonal antibody, a Fab fragment, a F(ab')₂ fragment, a V_{H} fragment, a V_{L} fragment, a Fv fragment, a single-chain variable fragment (scFv), a minibody, a diabody, a triabody, and a tetrabody. Preferably, the tau binding moiety recognizes an epitope within at least three contiguous amino acids of SEQ ID NO: 1, including within at least 6 contiguous amino acids of SEQ ID NO: 1, within at least 7 contiguous amino acids of SEQ ID NO: 1, within at least 8 contiguous amino acids of SEQ ID NO: 1, within at least 9 contiguous amino acids of SEQ ID NO: 1, within at least 10 contiguous amino acids of SEQ ID NO: 1, within at least 11 contiguous amino acids of SEQ ID NO: 1, within at least 12 contiguous amino acids of SEQ ID NO: 1, and within at least 13 contiguous amino acids of SEQ ID NO: 1, In an exemplary embodiment, a tau binding moiety of the present invention that recognizes an epitope within SEQ ID NO: 1 is the antibody HJ8.5, a humanized variant thereof, or a fragment thereof. In another exemplary embodiment, a tau binding moiety of the present invention that recognizes an epitope within SEQ ID NO: 1 is the antibody HJ8.1.1, a humanized variant thereof, or a fragment thereof. In preferred embodiments, the tau binding moiety is an scFv.

In another embodiment, a tau binding moiety of the present invention that specifically binds to tau recognizes an epitope within the amino acid sequence of SEQ ID NO: 2 (KTDHGAE), and is selected from the group consisting of a monoclonal antibody, a Fab fragment, a F(ab')₂ fragment, a V_{H} fragment, a V_{L} fragment, a Fv fragment, a single-chain variable fragment (scFv), a minibody, a diabody, a triabody, and a tetrabody. Preferably, the tau binding moiety recognizes an epitope within at least three contiguous amino acids of SEQ ID NO: 2, including within at least 4 contiguous amino acids of SEQ ID NO: 2 within at least 5 contiguous amino acids of SEQ ID NO: 2 within at least 6 contiguous amino acids of SEQ ID NO: 2, and within at least 7 contiguous amino acids of SEQ ID NO: 2. In an exemplary embodiment, a tau binding moiety of the present invention that recognizes an epitope within SEQ ID NO: 2 is the antibody HJ8.1.2, a humanized variant thereof, or a fragment thereof. In another exemplary embodiment, a tau binding moiety of the present invention that recognizes an epitope within SEQ ID NO: 2 is the antibody HJ8.4, a humanized variant thereof, or a fragment thereof. In preferred embodiments, the tau binding moiety is an scFv.

In another embodiment a tau binding moiety of the present invention that specifically binds to tau recognizes an epitope within the amino acid sequence of SEQ ID NO: 3 (PRHLSNV), and is selected from the group consisting of a monoclonal antibody, a Fab fragment, a F(ab')₂ fragment, a V_{H} fragment, a V_{L} fragment, a Fv fragment, a single-chain variable fragment (scFv), a minibody, a diabody, a triabody, and a tetrabody. Preferably, the tau binding moiety recognizes an epitope within at least three contiguous amino acids of SEQ ID NO: 3, including within at least 4 contiguous amino acids of SEQ ID NO: 3, within at least 5 contiguous amino acids of SEQ ID NO: 3, within at least 6 contiguous amino acids of SEQ ID NO: 3, and within at least 7 contiguous amino acids of SEQ ID NO: 3. In an exemplary embodiment, a tau binding moiety of the present invention that recognizes an epitope within SEQ ID NO: 3 is the antibody HJ8.2 or a humanized variant thereof. In another exemplary embodiment, a tau binding moiety of the present invention that recognizes an epitope within SEQ ID NO: 3 is the antibody HJ8.3, a humanized variant thereof, or a fragment thereof. In preferred embodiments, the tau binding moiety is an scFv.

In another embodiment, a tau binding moiety of the present invention that specifically binds to tau recognizes an epitope within the amino acid sequences of SEQ ID NO: 4 (EPRQ), and is selected from the group consisting of a monoclonal antibody, a Fab fragment, a F(ab')₂ fragment, a V_{H} fragment, a V_{L} fragment, a Fv fragment, a single-chain variable fragment (scFv), a minibody, a diabody, a triabody, and a tetrabody. Preferably, the tau binding moiety recognizes an epitope within at least three contiguous amino acids of SEQ ID NO: 4, including within at least 4 contiguous amino acids of SEQ ID NO: 4. In an exemplary embodiment, a tau binding moiety of the present invention that recognizes an epitope within SEQ ID NO: 4 is the antibody HJ8.8, a humanized variant thereof, or a fragment thereof. In preferred embodiments, the tau binding moiety is an scFv.

In another embodiment, a tau binding moiety of the present invention that specifically binds to tau recognizes an epitope within the amino acid sequence of SEQ ID NO: 5 (AAGHV), and is selected from the group consisting of a monoclonal antibody, a Fab fragment, a F(ab')₂ fragment, a V_{H} fragment, a V_{L} fragment, a Fv fragment, a single-chain variable fragment (scFv), a minibody, a diabody, a triabody, and a tetrabody. Preferably, the tau binding moiety recognizes an epitope within at least three contiguous amino acids of SEQ ID NO: 5, including within at least 4 contiguous amino acids of SEQ ID NO: 5, and within at least 5 contiguous amino acids of SEQ ID NO: 5. In an exemplary embodiment, a tau binding moiety of the present invention that recognizes an epitope within SEQ ID NO: 5 is the antibody HJ8.7, a humanized variant thereof, or a fragment thereof. In preferred embodiments, the tau binding moiety is an scFv.

In another embodiment, a tau binding moiety of the present invention that specifically binds to tau recognizes an epitope within the amino acid sequence of SEQ ID NO: 6 (TDHGAEIVYKSPVVSG), and is selected from the group consisting of a monoclonal antibody, a Fab fragment, a F(ab')₂ fragment, a V_{H} fragment, a V_{L} fragment, a Fv fragment, a single-chain variable fragment (scFv), a minibody, a diabody, a triabody, and a tetrabody. Preferably, the tau binding moiety recognizes an epitope within at least five contiguous amino acids of SEQ ID NO: 6, including within at least 6 contiguous amino acids of SEQ ID NO: 6, within at least 7 contiguous amino acids of SEQ ID NO: 6, within at least 8 contiguous amino acids of SEQ ID NO: 6, within at least 9 contiguous amino acids of SEQ ID NO: 6, within at least 9 contiguous amino acids of SEQ ID NO: 6, within at least 10 contiguous amino acids of SEQ ID NO: 6, within at least 11 contiguous amino acids of SEQ ID NO: 6, within at least 12 contiguous amino acids of SEQ ID NO: 6, within at least 13 contiguous amino acids of SEQ ID NO: 6, within at least 14 contiguous amino acids of SEQ ID NO: 6, within at least 15 contiguous amino acids of SEQ ID NO: 6, and within at least 16 contiguous amino acids of SEQ ID NO: 6. In an exemplary embodiment, a tau binding moiety of the present invention that recognizes an epitope within SEQ ID NO: 6 is the antibody HJ9.1, a humanized variant thereof, or a fragment thereof. In preferred embodiments, the tau binding moiety is an scFv.

In another embodiment, a tau binding moiety of the present invention that specifically binds to tau recognizes an epitope within the amino acid sequence of SEQ ID NO: 7 (EFEVMED), and is selected from the group consisting of a monoclonal antibody, a Fab fragment, a F(ab')₂ fragment, a V_{H} fragment, a V_{L} fragment, a Fv fragment, a single-chain variable fragment (scFv), a minibody, a diabody, a triabody, and a tetrabody. Preferably, the tau binding moiety recognizes an epitope within at least three contiguous amino acids of SEQ ID NO: 7, including within at least 4 contiguous amino acids of SEQ ID NO: 7, within at least 5 contiguous amino acids of SEQ ID NO: 7, within at least 6 contiguous amino acids of SEQ ID NO: 7, and within at least 7 contiguous amino acids of SEQ ID NO: 7. In an exemplary embodiment, an isolated antibody of the present invention that recognizes an epitope within SEQ ID NO: 7 is the antibody HJ9.2. In an exemplary embodiment, a tau binding moiety of the present invention that recognizes an epitope within SEQ ID NO: 7 is the antibody HJ9.4, a humanized variant thereof, or a fragment thereof. In an exemplary embodiment, a tau binding moiety of the present invention that recognizes an epitope within SEQ ID NO; 7 is the antibody HJ9.5, a humanized variant thereof, or a fragment thereof. In preferred embodiments, the tau binding moiety is an scFv.

In another embodiment, a tau binding moiety of the present invention that specifically binds to tau recognizes an epitope within the amino acid sequence of SEQ ID NO: 8 (GGKVQIINKK), and is selected from the group consisting of a monoclonal antibody, a Fab fragment, a F(ab')₂ fragment, a V_{H} fragment, a V_{L} fragment, a Fv fragment, a single-chain variable fragment (scFv), a minibody, a diabody, a triabody, and a tetrabody. Preferably, the tau binding moiety recognizes an epitope within at least three contiguous amino acids of SEQ ID NO: 8, including within at least 4 contiguous amino acids of SEQ ID NO: 8, within at least 5 contiguous amino acids of SEQ ID NO: 8, within at least 6 contiguous amino acids of SEQ ID NO: 8, within at least 7 contiguous amino acids of SEQ ID NO: 8, within at least 8 contiguous amino acids of SEQ ID NO: 8, within at least 9 contiguous amino acids of SEQ ID NO: 8, and within at least 10 contiguous amino acids of SEQ ID NO: 8. In an exemplary embodiment, a tau binding moiety of the present invention that recognizes an epitope within SEQ ID NO: 8 is the antibody HJ9.3, a humanized variant thereof, or a fragment thereof. In preferred embodiments, the tau binding moiety is an scFv.

In another embodiment, a tau binding moiety of the present invention is an antibody or fragment thereof derived from the antibody HJ8.5. As used herein, the term "derived from" means that the "derived" antibody comprises at least one CDR region from the antibody produced by hybridoma HJ8.5. Stated another way, the "derived antibody" comprises at least one CDR region comprised of the amino acid sequence selected from the group consisting of SEQ ID NO: 16,17, 18, 19, 20 and 21. An antibody or fragment thereof derived from the hybridoma HJ8.5 may be encoded by a nucleic acid sequence that has 90, 91, 92, 93, 94, 95, 96, 97, 98, or 99% identity to the light chain variable region of SEQ ID NQ:12; a nucleic acid that has 90, 91, 92, 93, 94, 95, 96, 97, 98, or 99% identity to the heavy chain variable region of SEQ ID NO:13; or a nucleic acid sequence that has 90, 91, 92, 93, 94, 95, 96, 97, 98, or 99% identity to the light chain variable region of SEQ ID NO:12 and 90, 91, 92, 93, 94, 95, 96, 97, 98, or 99% identity to the heavy chain variable region of SEQ ID NO:13. Alternatively, an antibody or fragment thereof derived from the hybridoma HJ8.5 may comprise an amino acid sequence that has 90, 91, 92, 93, 94, 95, 96, 97, 98, or 99% identity to the light chain variable region of SEQ ID NO:14; an amino acid sequence that has 90, 91, 92, 93, 94, 95, 96, 97, 98, or 99% identity to the heavy chain variable region of SEQ ID NO:15; or an amino acid sequence that has 90, 91, 92, 93, 94, 95, 96, 97, 98, or 99% identity to the light chain variable region of SEQ ID NO:14 and 90, 91, 92, 93, 94, 95, 96, 97, 98, or 99% identity to the heavy chain variable region of SEQ ID NO: 15. In each of the above embodiments, the antibody may be humanized. In preferred embodiments, the tau binding moiety is an scFv.

In an exemplary embodiment of a tau binding moiety of the invention that binds to tau, the tau binding moiety comprises the light chain nucleic acid sequence of SEQ ID NO:12 and the heavy chain nucleic add sequence of SEQ ID NO:13, In another exemplary embodiment of a tau binding moiety of the invention that binds to tau, the tau binding moiety comprises the light chain amino acid sequence of SEQ ID NO:14 and the heavy chain amino acid sequence of SEQ ID NO:15. In preferred embodiments, the tau binding moiety is an scFv.

In one embodiment a tau binding moiety of the invention may comprise a light chain CDR1, such as antibody 1 of Table B. In another embodiment, a tau binding moiety of the invention may comprise a light chain CDR2, such as antibody 5 of Table B. In yet another embodiment, a tau binding moiety of the invention may comprise a light chain CDR3, such as antibody 7 of Table B. In an alternative embodiment, a tau binding moiety of the invention may comprise a combination of two or three light chain CDRs, such as the antibodies 2, 3, 4 and 6 of Table B. For the avoidance of doubt, the term "antibody" includes "antibody fragments," as stated above. In preferred embodiments, the antibody is an scFv.

Similarly, in one embodiment, a tau binding moiety of the invention may comprise a heavy chain CDR1, such as antibody 8 of Table B. In another embodiment, a tau binding moiety of the invention may comprise a heavy chain CDR2, such as antibody 12 of Table B. In yet another embodiment, a tau binding moiety of the invention may comprise a heavy chain CDR3, such as antibody 14 of Table B. In an alternative embodiment, a tau binding moiety of the invention may comprise a combination of two or three heavy chain CDRs, such as the antibodies 9,10, 11, and 13 of Table B. For the avoidance of doubt, the term "antibody" includes "antibody fragments," as stated above. In preferred embodiments, the antibody is an scFv.

Alternatively, an antibody of the invention may comprise one or more light chain CDRs and one or more heavy chain CDRs, such as the antibodies 15-56 of **Table B**. For the avoidance of doubt, the term "antibody" includes "antibody fragments," as stated above. In preferred embodiments, the antibody is an scFv.

**Table B.**

| **Antibody** | **Light Chain** | | | **Heavy Chain** | | |
|---|---|---|---|---|---|---|
| | **CDR1** | **CDR2** | **CDR3** | **CDR1** | **CDR2** | **CDR3** |
| 1 | SEQ ID NO:16 | | | | | |
| 2 | SEQ ID NO:16 | SEQ ID NO:17 | | | | |
| 3 | SEQ ID NO:1 6 | | SEQ ID NO:18 | | | |
| 4 | SEQ ID NO: 16 | SEQ ID NO:17 | SEQ ID NO: 18 | | | |
| 5 | | SEQ ID NO: 17 | | | | |
| 6 | | SEQ ID NO:17 | SEQ ID NO:18 | | | |
| 7 | | | SEQ ID NO:18 | | | |
| 8 | | | | SEQ ID NO:19 | | |
| 9 | | | | SEQ ID NO:19 | SEQ ID NO:20 | |
| 10 | | | | SEQ ID NO:19 | | SEQ ID NO:21 |
| 11 | | | | SEQ ID NO: 19 | SEQ ID NO:20 | SEQ ID NO:21 |
| 12 | | | | | SEQ ID NO:20 | |
| 13 | | | | | SEQ ID NO:20 | SEQ ID NO:21 |
| 14 | | | | | | SEQ ID NO:21 |
| 15 | SEQ ID NO:16 | | | SEQ ID NO:19 | | |
| 16 | SEQ ID NO:16 | | | SEQ ID NO:19 | SEQ ID NO:20 | |
| 17 | SEQ ID NO:16 | | | SEQ ID NO:19 | | : SEQ ID NO:21 |
| 18 | SEQ ID NO:16 | | | SEQ ID NO:19 | SEQ ID NO:20 | SEQ ID NO:21 |
| 19 | SEQ ID NO:16 | | | | SEQ ID NO:20 | |
| 20 | SEQ ID NO:16 | | | | SEQ ID NO:20 | SEQ ID NO:21 |
| 21 | SEQ ID NO:16 | | | | | SEQ ID NO:21 |
| 22 | SEQ ID NO:16 | SEQ ID NO:17 | | SEQ ID NO:19 | | |
| 23 | SEQ ID NO:16 | SEQ ID NO: 17 | | SEQ ID NO: 19 | SEQ ID NO:20 | |
| 24 | SEQ ID NO:16 | SEQ ID NO:17 | | SEQ ID NO:19 | | SEQ ID NO:21 |
| 25 | SEQ ID NO:16 | SEQ ID NO:17 | | SEQ ID NO: 19 | SEQ ID NO:20 | SEQ ID NO:21 |
| 26 | SEQ ID NO:16 | SEQ ID NO:17 | | | SEQ ID NO:20 | |
| 27 | SEQ ID NO:16 | SEQ ID NO:17 | | | SEQ ID NO:20 | SEQ ID NO:21 |
| 28 | SEQ ID NO:16 | SEQ ID NO:17 | | | | SEQ ID NO:2 1 |
| 29 | SEQ ID NO:16 | SEQ ID NO: 17 | SEQ ID NO: 18 | SEQ ID NO:19 | | |
| 30 | SEQ ID NO:16 | SEQ ID NO: 17 | SEQ ID NO: 18 | SEQ ID NO:19 | SEQ ID NO:20 | |
| 31 | SEQ ID NO:16 | SEQ ID NO:17 | SEQ ID NO:18 | SEQ ID NO:19 | | SEQ ID NO:21 |
| 32 | SEQ ID NO:16 | SEQ ID NO:17 | SEQ ID NO:18 | SEQ ID NO:19 | SEQ ID NO:20 | SEQ ID NO:21 |
| 33 | SEQ ID NO:16 | SEQ ID NO:17 | SEQ ID NO:18 | | SEQ ID NO:20 | |
| 34 | SEQ ID NO:16 | SEQ ID NO:17 | SEQ ID NO:18 | | SEQ ID NO:20 | SEQ ID NO:21 |
| 35 | SEQ ID NO:16 | SEQ ID NO:17 | SEQ ID NO:18 | | | SEQ ID NO:21 |
| 36 | | SEQ ID NO:17 | | SEQ ID NO:19 | | |
| 37 | | SEQ ID NO:17 | | SEQ ID NO:19 | SEQ ID NO:20 | |
| 38 | | SEQ ID NO:17 | | SEQ ID NO:19 | | SEQ ID NO:21 |
| 39 | | SEQ ID NO:17 | | SEQ ID NO:19 | SEQ ID NO:20 | SEQ ID NO:21 |
| 40 | | SEQ ID NO: 17 | | | SEQ ID NO:20 | |
| 41 | | SEQ ID NO:17 | | | SEQ ID NO:20 | SEQ ID NO:21 |
| 42 | | SEQ ID NO:17 | | | | SEQ ID NO:21 |
| 43 | | SEQ ID NO:17 | SEQ ID NO:18 | SEQ ID NO:19 | | |
| 44 | | SEQ ID NO:17 | SEQ ID NO: 18 | SEQ ID NO:19 | SEQ ID NO:20 | |
| 45 | | SEQ ID NO.17 | SEQ ID NO:18 | SEQ ID NO:19 | | SEQ ID NO:21 |
| 46 | | SEQ ID NO:17 | SEQ ID NO:18 | SEQ ID NO:19 | SEQ ID NO:20 | SEQ ID NO:21 |
| 47 | | SEQ ID NO:17 | SEQ ID NO:18 | | SEQ ID NO 20 | |
| 48 | | SEQ 10 NO:17 | SEQ ID NO:18 | | SEQ ID NO:20 | SEQ ID NO:21 |
| 49 | | SEQ ID NO: 17 | SEQ ID NO:18 | | | SEQ ID NO:21 |
| 50 | | | SEQ ID NO:18 | SEQ ID NO:19 | | |
| 51 | | | SEQ ID NO:18 | SEQ ID NO:19 | SEQ ID NO:20 | |
| 52 | | | SEQ ID NO:18 | SEQ ID NO:19 | | SEQ ID NO:21 |
| 53 | | | SEQ ID NO:18 | SEQ ID NO:19 | SEQ ID NO:20 | SEQ ID NO:21 |
| 54 | | | SEQ ID NO:18 | | SEQ ID NO:20 | |
| 55 | | | SEQ ID NO:18 | | SEQ ID NO:20 | SEQ ID NO:21 |
| 56 | | | SEQ ID NO:18 | | | SEQ ID NO:21 |

In various embodiments, an antibody of the invention is humanized. For instance, in one embodiment, a humanized antibody of the invention may comprise a light chain variable region comprising a CDR1 of amino acid sequence SEQ ID NO: 16 with zero to two amino acid substitutions, a CDR2 of amino acid sequence SEQ ID NO: 17 with zero to two amino acid substitutions, and a CDR3 of amino acid sequence SEQ ID NO: 18 with zero to two amino acid substitutions, or may comprise a heavy chain variable region comprising a CDR1 of amino acid sequence SEQ ID NO: 19 with zero to two amino acid substitutions, a CDR2 of amino acid sequence SEQ ID NO: 20 with zero to two amino acid substitutions, and a CDR3 of amino acid sequence SEQ ID NO: 21 with zero to two amino acid substitutions. In a preferred embodiment, a humanized antibody of the invention may comprise a light chain variable region comprising a CDR1 of amino acid sequence SEQ ID NO: 16 with zero to two amino acid substitutions, a CDR2 of amino acid sequence SEQ ID NO: 17 with zero to two amino acid substitutions, a CDR3 of amino acid SEQ ID NO: 18 with zero to two amino acid substitutions, a heavy chain variable region comprising a CDR1 of amino acid sequence SEQ ID NO: 19 with zero to two amino acid substitutions, a CDR2 of amino acid sequence SEQ ID NO: 20 with zero to two amino acid substitutions, and a CDR3 of amino acid sequence SEQ ID NO: 21 with zero to two amino acid substitutions, In an exemplary embodiment, a humanized antibody of the invention may comprise a light chain variable region comprising a CDR1 of amino acid sequence SEQ ID NO: 16, a CDR2 of amino acid sequence SEQ ID NO: 17, a CDR3 of amino acid sequence SEQ ID NO: 18, a heavy chain variable region comprising a CDR1 of amino acid sequence SEQ ID NO: 19, a CDR2 of amino acid sequence SEQ ID NO: 20, and a CDR3 of amino acid sequence SEQ ID NO: 21. The invention also encompasses the corresponding nucleic acid sequences of SEQ ID NO: 16, 17, 18, 19, 20, and 21, which can readily be determined by one of skill in the art, and may be incorporated into a vector or other large DNA molecule, such as a chromosome, in order to express an antibody of the invention.

### II. Targeting Moiety

In an aspect, the present invention provides a targeting moiety. A targeting moiety refers to a polypeptide that is able to direct the entity to which it is attached (e.g., a tau binding moiety) to a target site.

In some embodiments, a targeting moiety may be capable of directing the entity to which it is attached to a receptor on the surface of celt that is capable of receptor-mediated endocytosis and lysosomal targeting. The targeting moiety may be an antibody or fragment thereof, an aptamer; or a binding domain derived from a target receptor ligand, In some embodiments, a targeting moiety may have an amino acid sequence comprising SEQ ID NO: 25 (TEELRVRLASHLRKLRKRLLRDA) or SEQ ID NO: 27 (SSVIDALQYKLEGTTRLTRKRGLKLATALSLSNKFVEGS), In other embodiments, a targeting moiety may have an amino acid sequence that has at least about 80%, at least about 85%, at least about 90%, at least about 95%, at least about 99% sequence identity to SEQ ID NO: 25 (TEELRVRLASHLRKLRKRLLRDA), or at least about 80%, at least about 85%, at least about 90%, at least about 95%, at least about 99% sequence identity to SEQ ID NO: 27 (SSVIDALQYKLEGTTRLTRKRGLKIATALSLSNKFVEGS). In other embodiments, a targeting moiety may have an amino acid sequence consisting essentially of SEQ ID NO: 25 (TEELRVRLASHLRKLRKRLLRDA), or SEQ ID NO: 27 (SSVIDALQYKLEDTTRLTRKRGLKLATALSLSNKFVEGS). In other embodiments, a polynucleotide sequence encoding a targeting moiety may comprise SEQ ID NO: 26 (ACCGAGGAGGTGCGGGTGCGCCTCGCCTCCCACCTGCGCAAGCTGCGTAAGCGGCTCC TCCGCGATGCC) or SEQ ID NO: 28 (TCATCTGTCATTGATGCACTGCAGTACAAATTAGAGGGCACCACAAGATTGACAAGAAAAA GGGGATTGAAGTTAGCCACAGCTCTGTCTCTGAGCAACAAATTTGTGGAGGGTAGT).

In some embodiments, a targeting moiety may be polypeptide sequence comprising the transmembrane and intracellular domain of a cell-surface receptor that is capable of receptor-mediated endocytosis and lysosomal targeting. Non-limiting examples of suitable cell-surface receptors include LDLR and LRP1. In some embodiments, a targeting moiety may have an amino acid sequence that comprises SEQ ID NO: 29. In other embodiments, a targeting moiety may have an amino acid sequence that has at least about 80%, at least about 85%, at least about 90%, at least about 95%, at least about 99% sequence identity to SEQ ID NO: 29. In other embodiments, a targeting moiety may have an amino acid sequence that consists essentially of SEQ ID NO: 29.

Other non-limiting examples of targeting moiety are binding motifs of a Heat Shock Cognate protein (HSC) that mediate chaperone-mediated autophagy; and ubiquitin or ubiquitin mutant, such as ubiquitin having a K48R point mutation for lysosomal degradation or a K63R point mutation for proteasomal degradation.

### III. Anti-tau construct

In an aspect, the present invention provides an anti-tau construct. An anti-tau construct of the invention is a polynucleotide sequence encoding a polypeptide, the polypeptide comprising at least one tau binding moiety and optionally comprising a signal peptide and/or a purification moiety. As used herein, the terms "polynucleotide sequence of the invention" and "anti-tau construct" are interchangeable. The present invention also provides isolated polypeptides encoded by anti-tau constructs, vectors comprising anti-tau constructs, and isolated cells comprising said vectors.

### (A) Polynticteotide sequence

An anti-tau construct of the invention is a polynucleotide sequence encoding a polypeptide, the polypeptide comprising at least one tau binding moiety and optionally comprising a signal peptide and/or a purification moiety, Accordingly, an anti-tau construct of the invention may be a polynucleotide sequence encoding a polypeptide, the polypeptide comprising 1, 2, 3, 4, 5, 6, 7, 8, 9, 10, or more tau binding moieties. Alternatively, an anti-tau construct of the invention may be a polynucleotide sequence encoding a polypeptide, the polypeptide comprising 1 to 10 tau binding moieties, 1 to 5 tau binding moieties, 5 to 10 tau binding moieties, 3 to 7 tau binding moieties, 1 to 3 tau binding moieties, or 3 to 5 tau binding moieties. One skilled in the art will appreciate that when two or more tau binding moieties are present, each tau binding moiety may specifically bind to the same or different antigenic determinant, in any number of combinations. As discussed above, a V_{H} fragment and a V_{L} fragment can be linked on the same polypeptide chain with a spacer stretching between the C-terminus of the first fragment to the N-terminus of the second fragment to create a scFv carrying a single antigen-binding site. However, in certain instances it may be desirable to create a polypeptide comprising two or more antigen-binding sites. For example, a polypeptide comprising two or more antigen-binding sites for the same antigenic determinant can result in the polypeptide binding to a multimeric antigen with greater avidity. As another example, a polypeptide comprising at least two different antigen-binding sites that specifically bind different antigenic determinants can allow the cross-linking of two antigens. Anti-tau constructs of the invention that are polynucleotide sequences encoding a polypeptide comprising two or more tau binding moieties, therefore, allow the polypeptide to be multivalent and/or be muitispecifc. Anti-tau constructs of the invention that are polynucleotide sequences encoding a polypeptide comprising one tau binding moiety have a single binding antigen-binding site.

In certain embodiments, the polynucleotide sequence of the invention may encode a polypeptide that further comprises a linker and a targeting moiety. The presence of a targeting moiety is not necessary, but may be included when it desirable to target tau bound by the tau binding moiety to a target site. As a non-limiting example, it may be desirable to target tau bound by a tau binding moiety to a cell for degradation. Without wishing to be bound by theory, it is believed this may be accomplished by several ways.

In some embodiments, an anti-tau construct of the invention is a polynucleotide sequence encoding a polypeptide, the polypeptide comprising at least one tau binding moiety attached via a linker to a targeting moiety, and optionally comprising a signal peptide and/or a purification moiety, wherein the targeting moiety is capable of directing the tau binding moiety to a receptor on the surface of cell that is capable of receptor-mediated endocytosis and lysosomal targeting.

In some embodiments, an anti-tau construct of the invention is a polynucleotide sequence encoding a polypeptide, the polypeptide comprising at least one tau binding moiety attached via a linker to a targeting moiety, and optionally comprising a signal peptide and/or a purification moiety, wherein the targeting moiety is selected from the group consisting of an antibody or fragment thereof, an aptamer, or a binding domain derived from a target receptor ligand, and the targeting moiety is capable of directing the tau binding moiety to a receptor on the surface of cell that is capable of receptor-mediated endocytosis and lysosomal targeting.

In some embodiments, an anti-tau construct of the invention is a polynucleotide sequence encoding a polypeptide, the polypeptide comprising at least one tau binding moiety attached via a linker to a targeting moiety, and optionally comprising a signal peptide and/or a purification moiety, wherein the targeting moiety is a binding domain derived from a target receptor ligand and the target receptor is the low-density lipoprotein receptor (LDLR) or the low-density lipoprotein receptor-related protein (LRP1). In an exemplary embodiment, the targeting moiety encoded by the polynucleotide may have an amino acid sequence comprising SEQ ID NO: 25 (TEELRVRLASHLRKLRKRLLRDA) or SEQ ID NO: 27 (SSVIDALQYKLEGTTRLTRKRGLKLATALSLSNKFVEGS). In another exemplary embodiment, the targeting moiety encoded by the polynucleotide may have an amino acid sequence that has at least about 70%, at least about 75%, at least about 80%, at least about 85%, at least about 90%, at least about 95%, at least about 99% sequence identity to SEQ ID NO: 25 (TEELRVRLASHLRMKRLLRDA) or at least about 70%, at least about 75%, at least about 80%, at least about 85%, at least about 90%, at least about 95%, at least about 99% sequence identity to SEQ ID NO: 27 (SSVIDALQYKLEGTTRLTRKRGLKLATALSLSNKFVEGS). In another exemplary embodiment, the targeting moiety encoded by the polynucleotide may have an amino acid sequence consisting essentially of SEQ ID NO: 25 (TEELRVRLASHLRKLRKRLLRDA) or SEQ ID NO: 27 (SSVIDALQYKLEGTTRLTRKRGLKLATALSLSNKFVEGS). In another exemplary embodiment, the polynucleotide sequence encoding the targeting moiety may comprise SEQ ID NO: 26 (ACCGAGGAGCTGCGGGTGCGCCTCGCCTCCCACCTGCGCAAGCTGCGTAAGCGGCTCC TCCGCGATGCC) or SEQ ID NO: 28 (TCATCTGTCATTGATGGACTGCAGTACAAATTAGAGGGCACCACAAGATTGACAAGAAAAA GGGGATTGÅAGTTAGCCACAGCTCTGTCTCTGAGCAACAAATTTGTGGAGGGTAGT).

In some embodiments, an anti-tau construct of the invention is a polynucleotide sequence encoding a polypeptide, the polypeptide comprising at least one tau binding moiety attached via a linker to a targeting moiety, and optionally comprising a signal peptide and/or a purification moiety, wherein the targeting moiety is a polypeptide sequence comprising the transmembrane and intracellular domain of a cell-surface receptor that is capable of receptor-mediated endocytosis and lysosomal targeting.

In some embodiments, an anti-tau construct of the invention is a polynucleotide sequence encoding a polypeptide, the polypeptide comprising at least one tau binding moiety attached via a linker to a targeting moiety, and optionally comprising a signal peptide and/or a purification moiety, wherein the targeting moiety is a polypeptide sequence comprising the transmembrane and intracellular domain of LDLR or LRP1. In an exemplary embodiment, the targeting moiety encoded by the polynucleotide may have an amino acid sequence that comprises SEQ ID NO: 29. In another exemplary embodiment, the targeting moiety encoded by the polynucleotide may have an amino acid sequence that has at least about 70%, at least about 75%, at least about 80%, at least about 85%, at least about 90%, at least about 95%, at least about 99% sequence identity to SEQ ID NO: 29. In another exemplary embodiment, the targeting moiety encoded by the polynucleotide may have an amino acid sequence that consists essentially of SEQ ID NO: 29. In another exemplary embodiment, the polynucleotide sequence encoding the targeting moiety may comprise SEQ ID NO: 30.

As described above in **Section II**, a tau binding moiety and a targeting moiety can be linked on the same polypeptide chain by a linker stretching between the C-terminus of the tau binding moiety to the N-terminus of the targeting moiety, or vice versa. Accordingly, in each of the above embodiments wherein a polynucleotide sequence encodes a polypeptide and the polypeptide comprises at least one tau binding moiety attached via a linker to a targeting moiety, the linker encoded by the polynucleotide may comprise an amino acid sequence of about 1 to 50 amino acid in length. In an exemplary embodiment, a spacer comprises the amino acid sequence (GGGS/T)ₙ or S/T(GGGS/T)ₙ, wherein *n* is an integer from 1 to 6, inclusive.

Each of the above embodiments may optionally comprise a signal peptide and/or a purification moiety. When present, typically the polynucleotide sequence encoding the signal peptide is at the N-terminus of the anti-tau construct and the polynucleotide sequence encoding the purification moiety is at the C-terminus of the anti-tau construct. The choice of polynucleotide sequence encoding the signal peptide can and will vary depending on a variety factors including, but not limited to, the desired cellular location and type of cell. Suitable polynucleotide sequence encoding signal peptides are known in the art, as polypeptide sequences encoded therefrom. In an exemplary embodiment, the polynucleotide sequence encoding the signal sequence may have a nucleic acid sequence that comprises SEQ ID NO: 22 (ATGGATATGAGAGTGCCTGCCCAACTTCTCGGACTGCTGCTGCTTTGGCTTAGAG GTGCAAGATGC) or SEQ ID NO: 24 (ATGCTGACCCCGCCGTTGCTCCTGCTOCTGCCCCTGCTCTCAGCTCTGGTCGCGGCGGC TATC). In another exemplary embodiment, the polynucleotide sequence encoding the signal sequence may have a nucleic acid sequence that has at least about 80%, at least about 85%, at least about 90%, at least about 95%, at least about 99% sequence identity to SEQ ID NO: 22 (ATGGATATGAGAGTGCCTGCCCAACTTCTCGGACTGCTGCTGCTTTGGCTTAGAG GTGCAAGATGC) or SEQ ID NO: 24 (ATGCTGACCCCGCCGTTGCTCCTGCTGCTGCCCCTGCTCTCAGCTCTGGTCGCGGCGGC TATC). In another exemplary embodiment, the polynucleotide sequence encoding the signal sequence may have a nucleic acid sequence that consists essentially of SEQ ID NO: 22 (ATGGATATGAGAGTGCCTGCCCAACTTCTCGGACTGCTGCTGCTTTGGCTTAGAG GTGCAAGATGC) or SEQ ID NO: 24 (ATGCTGACCCGGCCGTTGCTCCTGCTGCTGGCCCTGCTCTCAGCTCTGGTCGCGGCGGC TATC). In another exemplary embodiment, the signal peptide encoded by the polynucleotide may comprise SEQ ID NO: 21 (MDMRVPAQLLGLLLLWLRGARC), or SEQ ID NO: 23 (MLTPPLLLLLPLLSALVAAAIDAP). Similarly, the choice of purification moiety can and will vary. Suitable purification moieties are known in the art, as are the polynucleotide sequences encoding them, In an exemplary embodiment, the purification moiety encoded by the polynucleotide sequences of the invention may comprise SEQ ID NO: 31 (YPYDVPDYA),

In each of the above embodiments, a "tau binding moiety" may be as described in detail above in **Section I**, which is hereby incorporated by reference into this section. Preferably, in each of the above embodiments, the polynucleotide of the invention encodes a polypeptide, wherein the polypeptide comprises at least one tau binding moiety that is selected from the group consisting of: (i) a tau binding moiety that is a scFv, wherein the variable light chain of the scFv is encoded by a polynucleotide sequence comprising SEQ ID NO: 12 and the variable heavy chain of the scFv is encoded by a polynucleotide sequence comprising SEQ ID NO: 13; (ii) a tau binding moiety that is a scFv, wherein the scFv comprises SEQ ID NO: 14 and SEQ ID NO: 15, (iii) and a tau binding moiety that is a scFv, wherein the scFv comprises SEQ ID NO: 14 attached to SEQ ID NO: 15 via a polypeptide sequence, wherein the polypeptide sequence is (GGGS/T)ₙ or S/T(GGGS/T)ₙ, and *n* is an integer from 1 to 6, inclusive.

In some embodiments, an anti-tau construct of the invention is a polynucleotide sequence as depicted in **FIG. 1** or **FIG. 5**.

Anti-tau constructs of the invention may be produced from nucleic acids molecules using molecular biological methods known to in the art. Any of the methods known to one skilled in the art for the amplification of polynucleotide fragments and insertion of polynucleotide fragments into a vector may be used to construct the polynucleotide sequences of the invention. These methods may include *in vitro* recombinant DNA and synthetic techniques and *in vivo* recombinations (See Sambrook et al. Molecular Cloning, A Laboratory Manual, Cold Spring Harbor Laboratory; Current Protocols in Molecular Biology, Eds. Ausubel, et al., Greene Publ. Assoc., Wiley-interscience, NY).

In another aspect, the present invention provides a polynucleotide sequence encoding an anti-tau antibody, the antibody comprising a variable region and a constant region, wherein the variable region specifically binds an antigenic determinant of tau selected within an amino acid selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, and SEQ ID NO: 8, and the constant region is an IgG1, IgG2ab, IgG2abD265A, or IgG2b constant region. In certain embodiments, the variable region comprises a V_{L} fragment and the V_{L} fragment comprises a light chain CDR1 comprising the amino acid sequence of SEQ ID NO: 16 with zero to two amino acid substitutions, a light chain CDR2 comprising the amino acid sequence of SEQ ID NO: 17 with zero to two amino acid substitutions, a light chain CDR3 comprising the amino acid sequence of SEQ ID NO: 18 with zero to two amino acid substitutions, or any combination thereof. In certain embodiments, the variable region comprises a V_{H} fragment and the V_{H} fragment comprises a heavy chain CDR1 comprising the amino acid sequence of SEQ ID NO: 19 with zero to two amino acid substitutions, a heavy chain CDR2 comprising the amino acid sequence of SEQ ID NO: 20 with zero to two amino acid substitutions, a heavy chain CDR3 comprising the amino acid sequence of SEQ ID NO: 21 with zero to two amino acid substitutions, or any combination thereof.

### (B) Polypeptide sequence

In another aspect, the present invention provides an isolated polypeptide encoded by a polynucleotide sequence of the invention. Polynucleotide sequences of the invention are described in detail in Section III(a), and are hereby incorporated by reference into this section. An isolated polypeptide of the invention comprises at least one tau binding moiety and optionally comprises a signal peptide and/or a purification moiety. The isolated polypeptide may comprise 1, 2, 3, 4, 5, 6, 7, 8, 9, 10, or more tau binding moieties, 1 to 10 tau binding moieties, 1 to 5 tau binding moieties, 5 to 10 tau binding moieties, 3 to 7 tau binding moieties, 1 to 3 tau binding moieties, or 3 to 5 tau binding moieties. One skilled in the art will appreciate that when an isolated polypeptide of the invention comprises two or more tau binding moieties, each tau binding moiety may specifically bind to the same or different antigenic determinant, in any number of combinations,

In certain embodiments, a polypeptide of the invention may further comprise a targeting moiety attached to a tau binding moiety via a linker. In one embodiment, an isolated polypeptide may comprise at least one tau binding moiety attached to a targeting moiety via by a linker stretching between the C-terminus of the tau binding moiety to the N-terminus of the targeting moiety. In another embodiment, an isolated polypeptide may comprise at least one tau binding moiety attached to a targeting moiety via by a linker stretching between the C-terminus of the targeting moiety to the N-terminus of the tau binding moiety, In each of the above embodiments, the isolated polypeptide may comprise 1, 2, 3, 4, 5, 6, 7, 8, 9, 10, or more tau binding moieties, 1 to 10 tau binding moieties, 1 to 5 tau binding moieties, 5 to 10 tau binding moieties, 3 to 7 tau binding moieties, 1 to 3 tau binding moieties, or 3 to 5 tau binding moieties.

isolated polypeptides of the invention may be produced from nucleic acids molecules using molecular biological methods known to in the art. Generally speaking, a polynucleotide sequence encoding the polypeptide is inserted into a vector that is able to express the polypeptide when introduced into an appropriate host cell. Appropriate host cells include, but are not limited to, bacterial, yeast, insect, and mammalian cells. Once expressed, polypeptides may be obtained from cells of the invention using common purification methods. For example, if the polypeptide has a secretion signal, expressed polypeptides may be isolated from cell culture supernatant. Alternatively, polypeptides lacking a secretion signal may be purified from inclusion bodies and/or cell extract. Polypeptides of the invention may be isolated from culture supernatant, inclusion bodies or cell extract using any methods known to one of skill in the art, including for example, by chromatography (e.g., ion exchange, affinity, particularly by affinity for the specific antigen after Protein A, and sizing column chromatography), centrifugation, differential solubility, e.g. ammonium sulfate precipitation, or by any other standard technique for the purification of proteins; see, e.g., Scopes, "Protein Purification", Springer Verlag, N.Y. (1982). Isolation of polypeptides is greatly aided when the polypeptide comprises a purification moiety.

### (C) Vector

In another aspect, the present invention provides a vector comprising an anti-tau construct of the invention. As used herein, a vector is defined as a nucleic acid molecule used as a vehicle to transfer genetic material. Vectors include but are not limited to, plasmids, phasmids, cosmids, transposable elements, viruses (bacteriophage, animal viruses, and plant viruses), and artificial chromosomes (e.g., YACs), such as retroviral vectors (e.g. derived from Moloney murine leukemia virus vectors (MoMLV), MSCV, SFFV, MPSV,SNV etc), lentiviral vectors (e.g. derived from HIV-1, HIV-2, SIV, BIV, FIV etc.), adenoviral (Ad) vectors including replication competent, replication deficient and gutless forms thereof, adeno-associated viral (AAV) vectors, simian virus 40 (SV-40) vectors, bovine papilloma virus vectors, Epstein-Barr virus, herpes virus vectors, vaccinia virus vectors, Harvey murine sarcoma virus vectors, murine mammary tumor virus vectors, Rous sarcoma virus vectors.

In a specific embodiment, the vector is an expression vector. The vector may have a high copy number, an intermediate copy number, or a low copy number. The copy number may be utilized to control the expression level for the anti-tau construct, and as a means to control the expression vector's stability. In one embodiment, a high copy number vector may be utilized. A high copy number vector may have at least 31, 35, 40, 45, 50, 55, 60, 65, 70, 75, 80, 85, 90, 95, or 100 copies per bacterial cell. In other embodiments, the high copy number vector may have at least 100, 125, 150, 175, 200, 225, 250, 275, 300, 325, 350, 375, or 400 copies per host cell. In an alternative embodiment, a low copy number vector may be utilized. For example, a low copy number vector may have one or at least two, three, four, five, six, seven, eight, nine, or ten copies per host cell. In another embodiment, an Intermediate copy number vector may be used. For instance, an intermediate copy number vector may have at least 10, 11, 12, 13, 14, 15, 16, 17, 18, 19, 20, 21, 22, 23, 24, 25, 26, 27, 28, 29, or 30 copies per host cell.

Expression vectors typically contain one or more of the following elements- promoters, terminators, ribosomal binding sites, and IRES. Promoters that allow expression in all cell types such as the chicken beta actin promoter could be utilized. In addition cell type specific promoters for neurons (e.g. syapsin), astrocytes (e.g. GFAP), oligodendrocytes (e.g. myelin basic protein), or microglia (e.g. CX3CR1) could be used.

Expression of the nucleic acid molecules of the invention may be regulated by a second nucleic acid sequence so that the molecule is expressed in a host transformed with the recombinant DNA molecule. For example, expression of the nucleic acid molecules of the invention may be controlled by any promoter/enhancer element known in the art.

A nucleic acid encoding an anti-tau construct may also be operably linked to a nucleotide sequence encoding a selectable marker. A selectable marker may be used to efficiently select and identify cells that have integrated the exogenous nucleic acids. Selectable markers give the cell receiving the exogenous nucleic acid a selection advantage, such as resistance towards a certain toxin or antibiotic. Suitable examples of antibiotic resistance markers include, but are not limited to, those coding for proteins that impart resistance to kanamycin, spectomycin, neomycin, gentamycin (G418), ampicillin, tetracycline, chloramphenicol, puromycin, hygromycin, zeocin, and blasticidin.

In some embodiments, the vector may also comprise a transcription cassette for expressing reporter proteins. By way of example, reporter proteins may include a fluorescent protein, luciferase, alkaline phosphatase, beta-galactosidase, beta-lactamase, horseradish peroxidase, and variants thereof.

An expression vector encoding an anti-tau construct may be delivered to the cell using a viral vector or via a non-viral method of transfer. Viral vectors suitable for introducing nucleic acids into cells include retroviruses, adenoviruses, adeno-associated viruses, rhabdoviruses, and herpes viruses. Non-viral methods of nucleic acid transfer include naked nucleic acid, liposomes, and protein/nucleic acid conjugates. An expression construct encoding anti-tau construct that is introduced to the cell may be linear or circular, may be single-stranded or doublestranded, and may be DNA, RNA, or any modification or combination thereof.

An expression construct encoding an anti-tau construct may be introduced into the cell by transfection. Methods for transfecting nucleic acids are well known to persons skilled in the art, Transfection methods include, but are not limited to, viral transduction, cationic transfection, liposome transfection, dendrimer transfection, electroporation, heat shock, nucleofection transfection, magnetofection, nanoparticles, biolistic particle delivery (gene gun), and proprietary transfection reagents such as Lipofectamine, Dojindo Hilymax, Fugene, jetPEI, Effectene, or DreamFect.

Upon introduction into the cell. an expression construct encoding an anti-tau construct may be integrated into a chromosome. In some embodiments, integration of the expression construct encoding an anti-tau construct into a cellular chromosome may be achieved with a mobile element. The mobile element may be a transposon or a retroelement, A variety of transposons are suitable for use in the invention, Examples of DNA transposons that may be used include the Mu transposon, the P element transposons from Drosophila, and members of the Tc1/Mariner superfamily of transposons such as the sleeping beauty transposon from fish, A variety of retroelements are suitable for use in the invention and include LTR-containing retrotransposons and non-LTR retrotransposons. Non-limiting examples of retrotransposons include Copia and gypsy from Drosophila melanogaster, the Ty elements from Saccharomyces cerevisiae, the long interspersed elements (LINEs), and the short interspersed elements (SINEs) from eukaryotes. Suitable examples of LINEs include L1 from mammals and R2Bm from silkworm.

Integration of the exogenous nucleic acid into a cellular chromosome may also be mediated by a virus. Viruses that integrate nucleic acids into a chromosome include adeno-associated viruses and retroviruses. Adeno-associated virus (AAV) vectors may be from human or nonhuman primate AAV serotypes and variants thereof. Suitable adeno-associated viruses include AAV type 1, AAV type 2, AAV type 3, AAV type 4, AAV type 5, AAV type 6, AAV type 7, AAV type 8, AAV type 9, AAV type 10, and AAV type 11. A variety of retroviruses are suitable for use in the invention. Retroviral vectors may either be replication-competent or replication-defective. The retroviral vector may be an alpha retrovirus, a betaretrovirus, a gammaretrovirus, a deltaretrovirus, an epsilonretrovirus, a lentivirus, or a spumaretrovirus. In an embodiment, the retroviral vector may be a lentiviral vector. The lentiviral vector may be derived from human, simian, feline, equine, bovine, or lentiviruses that infect other mammalian species. Non-limiting examples of suitable lentiviruses includes human immunodeficiency virus (HIV), simian immunodeficiency virus (SIV), feline immunodeficiency virus (FIV), bovine immunodeficiency virus (BIV), and equine infectious anemia virus (EIAV)

Integration of an expression construct encoding an anti-tau construct into a chromosome of the cell may be random. Alternatively, integration of an expression construct encoding an anti-tau construct may be targeted to a particular sequence or location of a chromosome. In general, the general environment at the site of integration may affect whether the integrated expression construct encoding an anti-tau construct is expressed, as well as its level of expression. The virus may be altered to have tropism for a specific cell type. For example, the virus may be altered to have tropism for glial cells. Alternatively, the virus may be altered to have tropism for neuronal cells.

Cells transfected with the expression construct encoding an anti-tau construct generally will be grown under selection to isolate and expand cells in which the nucleic acid has integrated into a chromosome. Cells in which the expression construct encoding an anti-tau construct has been chromosomally integrated may be maintained by continuous selection with the selectable marker as described above. The presence and maintenance of the integrated exogenous nucleic acid sequence may be verified using standard techniques known to persons skilled in the art such as Southern blots, amplification of specific nucleic acid sequences using the polymerase chain reaction (PCR), and/or nucleotide sequencing.

Nucleic acid molecules are inserted into a vector that is able to express the fusion polypeptides when introduced into an appropriate host cell. Appropriate host cells include, but are not limited to, bacterial, yeast, insect, and mammalian cells.

In preferred embodiments, a vector-comprising an anti-tau construct of the invention is an adeno-associated viral (AAV) vector. Adeno-associated virus (AAV) is a replication-deficient parvovirus, the single-stranded DNA genome of which is about 4.7 kb in length including 145 nucleotide inverted terminal repeat (ITRs). The nucleotide sequence of the AAV serotype 2 (AAV2) genome is presented in Srivastava et al., J Virol, 45: 555-564 (1983) as corrected by Ruffing et al., J Gen Virol, 75: 3385-3392 (1994). Cis-acting sequences directing viral DNA replication, encapsidation/packaging and host cell chromosome integration are contained within the ITRs. Three AAV promoters (named p5, p19, and p40 for their relative map locations) drive the expression of the two AAV internal open reading frames encoding rep and cap genes. The two rep promoters (p5 and p19), coupled with the differential splicing of the single AAV intron (at nucleotides 2107 and 2227), result in the production of four rep proteins (rep 78, rep 68, rep 52, and rep 40) from the rep gene. Rep proteins possess multiple enzymatic properties that are ultimately responsible for replicating the viral genome. The cap gene is expressed from the p40 promoter and it encodes the three capsid proteins VP1, VP2, and VP3. Alternative splicing and non-consensus translational start sites are responsible for the production of the three related capsid proteins. A single consensus polyadenylation site is located at map position 95 of the AAV genome. The life cycle and genetics of AAV are reviewed in Muzyczka, Current Topics in Microbiology and Immunology, 158: 97-129 (1992).

AAV possesses unique features that make it attractive as a vector for delivering foreign DNA to cells, for example, in gene therapy. AAV infection of cells in culture is noncytopathic, and natural infection of humans and other animals is silent and asymptomatic. Moreover, AAV infects many mammalian cells allowing the possibility of targeting many different tissues in vivo. Moreover, AAV transduces slowly dividing and non-dividing cells, and can persist essentially for the lifetime of those cells as a transcriptionally active nuclear episome (extrachromosomal element). Furthermore, because the signals directing AAV replication, genome encapsidation and integration are contained within the ITRs of the AAV genome, some or all of the internal approximately 4.3 kb of the genome (encoding replication and structural capsid proteins, rep-cap) may be replaced with foreign DNA such as a gene cassette containing a promoter, a DNA of interest and a polyadenylation signal. The rep and cap proteins may be provided in trans. Another significant feature of AAV is that it is an extremely stable and hearty virus. It easily withstands the conditions used to inactivate adenovirus, making cold preservation of AAV less critical. AAV may even be lyophilized. Finally, AAV-infected cells are not resistant to superinfection.

Multiple serotypes of AAV exist and offer varied tissue tropism. Known serotypes include, for example, AAV1, AAV2, AAV3, AAV4, AAV5, AAV6, AAV7, AAV8, AAV9, AAV10 and AAV11. AAV9 is described in U.S. Pat. No. 7,198,951 and in Gao et al., J. Virol., 78: 6381-6388 (2004). Advances in the delivery of AAV6 and AAV8 have made possible the transduction by these serotypes of skeletal and cardiac muscle following simple systemic intravenous or intraperitoneal injections. See, Pacak et al., Circ Res 99(4): 3-9 (1006) and Wang et al., Nature Biotech,23(3): 321-328 (2005). The use of some serotypes of AAV to target cell types within the central nervous system, though, has required surgical intraparenchymal injection. See, Kaplitt et al, Lancet 369 2097-2105 (2007); Marks et al., Lancet Neurol 7: 400-408 (2008); and Worgall et al.,Hum Gene Ther (2008).

An adeno-associated viral (AAV) vector is a plasmid comprising a recombinant AAV genome. The DNA plasmids are transferred to cells permissible for infection with a helper virus of AAV (e.g., adenovirus, E1-deleted adenovirus or herpesvirus) for assembly of the rAAV genome into infectious viral particles. Techniques to produce rAAV particles, in which an AAV genome to be packaged, rep and cap genes, and helper virus functions are provided to a cell are standard in the art Production of rAAV requires that the following components are present within a single cell (denoted herein as a packaging cell): a rAAV genome, AAV rep and cap genes separate from (i.e., not in) the rAAV genome, and helper virus functions. The AAV rep and cap genes may be from any AAV serotype for which recombinant virus can be derived and may be from a different AAV serotype than the rAAV genome ITRs, including, but not limited to, AAV serotypes AAV-1, AAV-2, AAV-3, AAV-4, AAV-5, AAV-6, AAV-7, AAV-8, AAV-9, AAV-10 and AAV-11. Production of pseudotyped rAAV is disclosed in, for example, WO 01/83692 which is incorporated by reference herein in its entirety. In an exemplary embodiment, a vector is based on the AAV2 serotype. In another exemplary embodiment, a vector is based on the AAV9 serotype (see, for example, Foust et al., Nature Biofechnology,27: 59-65 (2009); Duque et al., Mol. Ther. 17: 1187-1196 (2009); Zincarelliet al., Mol. Ther., 16: 1073-1080 (2008); and U.S. Patent Publication No. 20130039888).

A method of generating a packaging cell is to create a cell line that stably expresses all the necessary components for AAV particle production. For example, a plasmid (or multiple plasmids) comprising a rAAV genome lacking AAV rep and cap genes, AAV rep and cap genes separate from the rAAV genome, and a selectable marker, such as a neomycin resistance gene, are integrated into the genome of a cell. AAV genomes have been introduced into bacterial plasmids by procedures such as GC tailing (Samulski et al., 1982, Proc. Natl. Acad. S6. USA, 79:2077-2081), addition of synthetic linkers containing restriction endonuclease cleavage sites (Laughlin et al., 1983, Gene, 23:65-73) or by direct, blunt-end ligation (Senapathy & Carter, 1984, J. Biol. Chem., 259:4661-4866). The packaging cell line is then infected with a helper virus such as adenovirus, The advantages of this method are that the cells are selectable and are suitable for large-scale production of rAAV. Other examples of suitable methods employ adenovirus or baculovirus rather than plasmids to introduce rAAV genomes and/or rep and cap genes into packaging cells.

General principles of rAAV production are reviewed in, for example, Carter, 1992, Current Opinions in Biotechnology, 1533-539; and Muzyczka, 1992, Curr. Topics in Microbial, and Immunol, 158:97-129). Various approaches are described in Ratschin et al., Mol. Cell. Biol. 4:2072 (1984); Hermonat et al., Proc. Natl. Acad. Sci USA, 81:6466 (1984); Tratschin et al., Mol. Cell. Biol 5:3251 (1985); McLaughlin et al., J. Virol., 62:1963 (1988); and Lebkowski et al., 1 988 Mol. Cell. Biol., 7:349 (1988). Samulski et al. (1989, J. Virol., 63:3822-3828); U.S. Pat. No. 5,173,414; WO 95/13365 and corresponding U.S. Pat. No. 5,658,776; WO 95/13392; WO 96/17947; PCT/US98/18600; WO 97/09441 (PCT/US96/14423); WO 97/08298 (PCT/US96/13872); WO 97/21825 (PCT/US96/20777); WO 97/06243 (PCT/FR96/01064);WO 99/11764; Perrin et al. (1995) Vaccine 13:1244-1250; Paul et al. (1993) Human Gene Therapy 4:609-615; Clark et al (1996) Gene Therapy 3:1124-1132; U.S. Pat. No. 5,786,211; U.S. Pat. No. 5,871,982; and U.S. Pat. No. 6,258,595. The foregoing documents are hereby incorporated by reference in their entirety herein, with particular emphasis on those sections of the documents relating to rAAV production,

The invention thus provides packaging cells that produce infectious rAAV.

In another aspect, the invention provides rAAV (i.e., infectious encapsidated rAAV particles) comprising a rAAV genome of the invention. In some embodiments of the invention, the rAAV genome is a self-complementary genome,

### (C)Isolated Cell

In another aspect, the present invention provides an isolated cell comprising a vector of the invention. The cell may be a prokaryotic cell or a eukaryotic cell. Appropriate cells include, but are not limited to, bacterial, yeast, insect, and mammalian cells,

In some embodiments, the isolated host cell comprising a vector of the invention may be used to produce a polypeptide encoded by an anti-tau construct of the invention. Generally, production of a polypeptide of the invention involves transfecting isolated host cells with a vector comprising an anti-tau construct and then culturing the cells so that they transcribe and translate the desired polypeptide, The isolated host cells may then be lysed to extract the expressed polypeptide for subsequent purification, "Isolated host cells" according to the invention are cells which have been removed from an organism and/or are maintained *in vitro* in substantially pure cultures. A wide variety of cell types can be used as isolated host cells of the invention, including both prokaryotic and eukaryotic cells. Isolated cells include, without limitation, bacterial cells, fungal cells, yeast cells, insect cells, and mammalian cells.

In one embodiment, the isolated host cell is characterized in that after transformation with a vector of the invention, it produces the desired polypeptide for subsequent purification. Such a system may be used for protein expression and purification as is standard in the art. In some embodiments, the host cell is a prokaryotic cell. Non-limiting examples of suitable prokaryotic cells include *E. coli* and other Enterobacteriaceae, *Escherichia* sp., *Campylobacter* sp., *Wolinella* sp., *Desulfovibrio* sp. *Vibrio* sp., *Pseudomonas* sp. *Bacillus* sp., *Listeria* sp., *Staphylococcus* sp., *Streptococcus* sp., *Peptostreptococcus* sp., *Megasphaera* sp., *Pectinatus* sp., *Selenomonas* sp., *Zymophilus* sp., *Actinomyces* sp., *Arthrobacter* sp., *Frankia* sp., *Micromonospora* sp., *Nocardia* sp., *Propionibacterium* sp., *Streptomyces* sp., *Lactobacillus sp., Lactococcus* sp., *Leuconostoc* sp., *Pediococcus* sp., *Acetobacterium* sp., *Eubacterium* sp., *Heliobacterium* sp., *Heliospirillum* sp., *Sporomusa* sp., *Spiroplasma* sp., *Ureaplasma* sp., *Erysipelothrix* sp., *Corynebacterium* sp. *Enterococcus* sp., *Clostridium* sp., *Mycoplasma* sp., *Mycobacterium* sp., *Actinobactería* sp., *Salmonella* sp., *Shigella* sp., *Moraxella* sp., *Helicobacter* sp, *Stenotrophomonas* sp., *Micrococcus* sp., *Neisseria* sp., *Bdellovibrio* sp., *Hemophilus* sp., *Klebsiella* sp., *Proteus mirabilis, Enterobacter cloacae, Serratia* sp., *Citrobacter* sp., *Proteus* sp., *Serratia* sp., *Yersinia* sp., *Acinetobacter* sp.*, Actinobacillus* sp. *Bordetella* sp., *Brucella* sp., *Capnocytophaga* sp., *Cardiobacterium* sp., *Eikenella* sp., *Francisella* sp., *Haemophilus* sp., *Kingella* sp., *Pasteurella* sp., *Flavobacterium* sp. *Xanthomonas* sp., *Burkholderia* sp., *Aeromonas* sp., *Plesiomonas* sp., *Legionella* sp. and alpha-proteobacteria such as *Wolbachia* sp., cyanobacteria, spirochaetes, green sulfur and green non-sulfur bacteria, Gram-negative cocci, Gram negative bacilli which are fastidious, Enterobacteriaceae-glucose-fermenting gram-negative bacilli, Gram negative bacilli-non-glucose fermenters, Gram negative bacilli-glucose fermenting, oxidase positive.

Particularly useful bacterial host cells for protein expression include Gram negative bacteria, such as *Escherichia coli, Pseudomonas fluorescens, Pseudomonas haloplanctis, Pseudomonas putida* AC10, *Pseudomonas pseudoflava, Bartonella henselae, Pseudomonas syringae, Caulobacter crescentus, Zymomonas mobilis, Rhizobium meliloti, Myxococcus xanthus* and Gram positive bacteria such as *Bacillus subtilis, Corynebacterium, Streptococcus cremoris, Streptococcus lividans,* and *Streptomyces lividans. E coli* is one of the most widely used expression hosts. Accordingly, the techniques for overexpression in *E. coli* are well developed and readily available to one of skill in the art Further, *Pseudomonas fluorescens,* is commonly used for high level production of recombinant proteins (i.e. for the development biotherapeutics and vaccines).

Particularly useful fungal host celts for protein expression include *Aspergillis oryzae, Aspergillis niger, Trichoderma reesei, Aspergillus nidulans, Fusarium graminearum.*

Particularly useful yeast host cells for protein expression include *Candida albicans, Candida maltose, Hansenula polymorpha, Kluyveromyces fragilis, Kluyveromyces lactis, Pichia guillerimondii, Pichia pastoris, Saccharomyces cerevisiae, Schizosaccharomyces pombe,* and *Yarrowia lipolytica.*

Particularly useful mammalian host cells for protein expression include Chinese hamster ovary (CHO) cells, HeLa cells, baby hamster kidney (BHK) cells, monkey kidney cells (COS), human hepatocellular carcinoma cells (eg. Hep G2), human embryonic kidney cells, *Bos primigenius,* and *Mus musculus.* Additionally, the mammalian host cell may be an established, commercially-available cell line (e.g., American Type Culture Collection (ATCC), Manassas, VA). The host cell may be an immortalized cell. Alternatively, the host cell may be a primary cell. "Primary cells" are cells taken directly from living tissue (i.e. biopsy material) and established for growth *in vitro,* that have undergone very few population doublings and are therefore more representative of the main functional components and characteristics of tissues from which they are derived from, in comparison to continuous tumorigenic or artificially immortalized cell lines.

In another embodiment, the host cell may be *in vivo,* i.e., the cell may be disposed in a subject. Accordingly, a polypeptide of the invention is expressed from a host cell in the subject. In certain embodiments, a host cell in a subject may be selected from the group consisting of neurons, astrocytes, oligodendrocytes, microglia, chroroid plexus cells, brain blood vessel endothelial cells, brain blood vessel smooth muscle cells, and brain blood vessel pericytes. In a specific embodiment, the host cell may be neuronal or glial cell. In an exemplary embodiment, an AAV vector may be used to express a polypeptide of the invention in a host cell disposed in a subject.

### IV. Methods

In another aspect, the present invention provides a method of delivering an anti-tau construct of the invention to a cell. In some embodiments, the method comprises contacting the cell with a composition comprising a vector, the vector comprising an anti-tau construct of the invention. In other embodiments, the method comprises contacting the cell with a composition comprising a cell, the cell comprising an anti-tau construct of the invention. In other embodiments, the method comprises contacting a cell with a composition comprising a rAAV. the rAAV comprising an anti-tau construct of the invention. In preferred embodiments, the anti-tau construct is a polynucleotide sequence encoding a polypeptide comprising a signal peptide, and at least one tau binding moiety attached via a linker to a targeting moiety. In each of the above embodiments, a composition may further comprise an excipient. Non-limiting examples of excipients include antioxidants, binders, buffers, diluents (fillers), disintegrants, dyes, effervescent disintegration agents, preservatives (antioxidants), flavor-modifying agents, lubricants and glidants, dispersants, coloring agents, pH modifiers, chelating agents, preservatives (e.g., antibacterial agents, antifungal agents), release-controlling polymers, solvents, surfactants, and combinations of any of these agents. Cells are contacted with the composition comprising a vector or protein of the invention under effective conditions for a period of time sufficient to deliver an anti-tau construct to a cell, Suitable cells are described above in **Section III**, and hereby incorporated by reference into this section. For example, the cell may be a bacterial cell, a yeast cell, an insect celt, or a mammalian cell. The choice of cells can and will vary depending upon the goal.

In certain embodiments, the goal may be to obtain isolated polypeptide of the invention. Cell types for protein production are well known in the art and a suitable cell type can be readily selected by one of skill in the art.

In certain embodiments, the goal may be to deliver an anti-tau construct to a cell of a subject. The subject's cell may be isolated, or the anti-tau construct may be delivered to the cell in the subject. Any cell type may be targeted. In preferred embodiments, the cell is a mammalian ceil selected from the group consisting of neurons, astrocytes, oligodendrocytes, microglia, chroroid plexus cells, brain blood vessel endothelial cells, brain blood vessel smooth muscle cells, and brain blood vessel pericytes. When the subject's cell is not isolated, the composition may be administered to the subject orally, parenteraly, intraperitoneally, intravascularly, intrapulmonary, or topically. The term parenteral as used herein includes subcutaneous, intravenous, intramuscular, intrathecal, or intrasternal injection, or infusion techniques.

In another aspect, the present invention provides a method of delivering a tau binding moiety to, and/or or targeting tau in, an intracellular vesicle in a subject. In some embodiments, the method comprises administering to the subject a composition comprising an anti-tau construct of the invention. In some embodiments, the method comprises administering to the subject a composition comprising a vector, the vector comprising an anti-tau construct of the invention. In other embodiments, the method comprises administering to the subject a composition comprising an isolated polypeptide encoded by an anti-tau construct of the invention. In other embodiments, the method comprises administering to the subject a composition comprising a cell, the cell comprising an anti-tau construct of the invention. In other embodiments, the method comprises administering to the subject a composition comprising a rAAV, the rAAV comprising an anti-tau construct of the invention. In preferred embodiments, the anti-tau construct is a polynucleotide sequence encoding a polypeptide comprising a signal peptide, and at least one tau binding moiety attached via a linker to a targeting moiety. The composition may be administered to the subject orally, parenteraly, intraperitoneally, intravascularly, intrapulmonary, or topicaliy. Suitable subjects are described above, and hereby incorporated by reference into this section. In preferred embodiments, a subject may be a laboratory animal or a human. In each of the above embodiments, a composition may further comprise an excipient. Non-limiting examples of excipients include antioxidants, binders, buffers, diluents (fillers), disintegrants, dyes, effervescent disintegration agents, preservatives (antioxidants), flavor-modifying agents, lubricants and glidants, dispersants, coloring agents, pH modifiers, chelating agents, preservatives (e.g., antibacterial agents, antifungal agents), release-controlling polymers, solvents, surfactants, and combinations of any of these agents.

In another aspect, the present invention provides a method of delivering an anti-tau antibody to an intracellular vesicle in a subject, the method comprising administering to the subject a composition comprising a composition or vector comprising the polynucleotide encoding an anti-tau antibody. In certain embodiments, the antibody comprises a variable region and a constant region, wherein the variable region specifically binds an antigenic determinant of tau selected within an amino acid selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, and SEQ lD NO: 8, and the constant region is an IgG1, IgG2ab, IgG2abD265A, or IgG2b constant region. In certain embodiments, the variable region comprises a V_{L} fragment and the V_{L} fragment comprises a light chain CDR1 comprising the amino acid sequence of SEQ ID NO: 16 with zero to two amino acid substitutions, a light chain CDR2 comprising the amino acid sequence of SEQ ID NO: 17 with zero to two amino acid substitutions, a light chain CDR3 comprising the amino acid sequence of SEQ ID NO: 18 with zero to two amino acid substitutions, or any combination thereof. In certain embodiments, the variable region comprises a V_{H} fragment and the V_{H} fragment comprises a heavy chain CDR1 comprising the amino acid sequence of SEQ ID NO: 19 with zero to two amino acid substitutions, a heavy chain CDR2 comprising the amino acid sequence of SEQ ID NO: 20 with zero to two amino acid substitutions, a heavy chain CDR3 comprising the amino acid sequence of SEQ ID NO: 21 with zero to two amino acid substitutions, or any combination thereof. In certain embodiments, the vector is an AAV vector, for example a recombinant AAV vector.

When administering a composition comprising an rAAV. one of skill in the art will appreciate that the method of the administration will vary depending, for example, on the particular rAAV and the cell type(s) being targeted, and may be determined by methods standard in the art. Titers of rAAV may range from about 1×10⁶, about 1×10⁷, about 1×10⁸, about 1×10⁹, about 1×10¹⁰, about 1×10¹¹, about 1×10¹², about 1×10¹³to about 1×10¹⁴ or more DNase resistant particles (DRP) per ml. Dosages may also be expressed in units of viral genomes (vg). Dosages may also vary based on the timing of the administration to a human. These dosages of rAAV may range from about 1×10¹¹ vg/kg, about 1 ×10¹², about 1×10¹³, about 1×10¹⁴, about 1×10¹⁵, about 1 × 10¹⁶ or more viral genomes per kilogram body weight in an adult. For a neonate, the dosages of rAAV may range from about 1 ×10¹¹, about 1×10¹², about 3×10¹², about 1×10¹³, about 3×10¹³, about 1×10¹⁴, about 3×10¹⁴, about 1×10¹⁵, about 3×10¹⁵, about 1 ×10¹⁶, about 3x 10¹⁶ or more viral genomes per kilogram body weight.

The following examples are included to demonstrate preferred embodiments of the invention. It should be appreciated by those of skill in the art that the techniques disclosed in the examples that follow represent techniques discovered by the inventors to function well in the practice of the invention. Those of skill in the art should, however, in light of the present disclosure, appreciate that changes may be made in the specific embodiments that are disclosed and still obtain a like or similar result without departing from the spirit and scope of the invention. Therefore, all matter set forth or shown in the accompanying drawings is to be interpreted as illustrative and not in a limiting sense.

### EXAMPLES

The following examples illustrate various iterations of the invention.

### Example 1. scFv's of anti-tau antibody HJ8.5 are expressed, secreted and readily bind human tau.

The anti-tau antibodies disclosed in Table A have been described elsewhere. Further development of the anti-tau antibodies, and specifically HJ8.5, has resulted in the creation of single chain variable fragments (scFv) of the anti-tau antibodies. Specifically, scFv derivatives of HJ8,5 were developed with varying spacer length. **FIG. 1** depicts three exemplary scFv derivatives of HJ8.5, The scFv's comprise a secretory signal peptide (SP) for secretion of the protein, the variable region light chain (V_{L}) of HJ8.5, a spacer, the variable region heavy chain (V_{H}) of HJ8,5 and an HA-tag (HA). Although **FIG. 1** depicts the V_{L} first and the V_{H} second, it is contemplated that the V_{H} could be first and the V_{L} could be second. Further, the signal peptide and HA-tag are not required. The three scFV's depicted have varying spacer lengths. SC1 comprises the spacer (GGGS)_{1,} SC2 comprises the spacer S(GGGS)₂, and SC3 comprises the spacer S(GGGS)₃. Additional spacers may be used.

It was necessary to confirm that the scFv's were both efficiently secreted and bound to tau. HEK 293 cells were transfected with the three constructs (encoding SG1, SC2 and SC3). After 48 hours, the cell culture supernatant was collected. Additionally, whole cell lysate was collected and prepared to analyze the expression of the constructs and the amount retained in the cell. **FIG. 2** (bottom panel) shows an anti-HA Western blot to detect scFv present in the whole cell lysate. It is evident that all three constructs are readily expressed. Recombinant human tau was added to the collected supernatant. SC1, SC2 and SC3 were then immunoprecipitated from the supernatant using an anti-HA antibody. **FIG. 2** (middle panel) shows that all SC1, SC2 and SC3 can be readily detected in the supernatant. Importantly, **FIG. 2** (top panel) shows that SC1, SC2 and SC3 bind to tau in solution. Immunoprecipitation of SC1, SC2 and SC3 resulted in the ability to detect tau by Western blot confirming that the scFv's bound to the tau added to the supernatant

To further confirm the binding of the HJ8.5 scFv's to tau, a tau-ELISA was performed. The HJ8,5 scFv's were expressed in HEK 293 cells and purified from the supernatant using an anti-HA agarose column. Recombinant human tau was added to a ELISA plate at 100 µg/ml. The HJ8.5 scFv's (e.g. SC1, SC2 and SC3) were added to the wells contain tau and an anti-HA antibody coupled to HRP was used to detect binding of the scFv's to tau. FIG. 3 shows that all three scFv's bound to tau.

It is known that monoclonal antibody HJ8,5 binds to human tau but not mouse tau. To confirm this feature is also observed with the scFv's, the binding of SC1 and SC3 to mouse and human tau was evaluated. **FIG. 4** shows that as with the parent antibody, SC1 and SC3 bind to recombinant human tau but do not bind to mouse tau. In summary, the results show that the HJ8.5 scFv's tested are expressed, secreted into the supernatant and specifically bind to human tau.

### Example 2. Development of scFv's useful to target tau to LDLR/LRP1-mediated cellular clearance

Receptor-mediated endocytosis in neurons by the low-density lipoprotein receptor-related protein 1 (LRP1) plays a critical rote in brain Aβ clearance, LRP1 is known to be an endocytic receptor for multiple ligands including Aβ. Apolipoprotein B (ApoB) and apolipoprotein E (ApoE) have binding domains for LRP1. Accordingly, an scFv with the ability to bind tau comprising LRP1 binding affinity (i.e. a receptor binding domain from ApoB or apoE) may enhance the efficacy of tau clearance by targeting the scFv bound to tau to the endosome via LRP1. Based on this, HJ8.5 scFv's were developed further comprising a receptor-binding domain from ApoB or ApoE. The receptor binding domain from ApoB or ApoE targets the construct to an endosome expressing LRP1. FIG. 5 depicts exemplary constructs of SC1 or SC3 further comprising a linker and a LRP1 binding domain (i.e. from ApoB or ApoE). The scFv's comprise a secretory signal peptide (SP), a variable region light chain (V_{L}), a spacer, a variable region heavy chain (V_{H}), a linker, the receptor-binding domain of ApoB or ApoE (ApoB-BD or ApoE-BD) and an HA-tag (HA). SC1 comprises the spacer (GGGS)₁; and SC3 comprises the spacer S(GGGS)₃. The linker comprises the sequence S(GGGS)₄. The linker is prone for degradation in lysosomes. The receptor binding domain of ApoB and/or ApoE may be used to target the polypeptide, and the polypeptide bound to tau, for LDLR/LRP1-mediated cellular clearance.

It was necessary to confirm that the scFv's were efficiently secreted. HEK 293 cells were transfected with the six constructs (encoding SC1 and SC3 without apoB/E-BD and SC1 and SC3 comprising apoB-BD or apoE-BD). The cell culture supernatant was collected. FIG.6 shows that all six scFv's can be readily detected in the supernatant indicating efficient expression of the various constructs.

It was also evaluated if the constructs could be expressed and secreted using an AAV vector, **FIG. 7** (top panel) shows that all 6 constructs are expressed and secreted into the supernatant using either the pcDNA3 plasmid or an AAV vector. **FIG. 7** (bottom panel) shows that all 6 constructs are expressed and the polypeptides encoded by the constructs are present in the whole cell lysate when using either the pcDNA3 plasmid or an AAV vector. Importantly, these results illustrate that the various constructs can be expressed and the resultant polypeptide secreted using an AAV vector.

As another means to target the scFv_{'}s for LRP1-mediated clearance, the scFv's were fused with the transmembrane and intracellular domain of LRP1. This allows for tau to be bound to an scFv which is in turn fused to the LRP1 protein found on endosomes to potentially enhance clearance of tau. **FIG. 8A** depicts a schematic of the anti-tau scFv-LRP1 construct **FIG. 8B** shows that the LRP1 scFv is expressed and binds tau (see lane 4). Further, **FIG. 8C** shows that varying linker lengths may be used to enhance tau clearing from cell culture media. In summary, it has successfully been shown that scFv's designed to target tau to the endosomal compartment can be generated and expressed and also retain binding affinity for tau.

### Example 3. AAV constructs can be successfully expressed in vivo

It has been successfully shown that scFv's may be expressed in *vitro* using an AAV vector. To confirm that this vector will also readily express protein *in* vivo, constructs were constructed as depicted in **FIG. 9****.** A control construct and a Tau P301S construct were generated. The control construct comprises GAGS promoter, IRE and GFP and the Tau P301S construct comprises CAGS promoter, Tau, IRE and GFP. The constructs may be used to express proteins in brain for rapid assessment. **FIG. 10** shows that these constructs are successfully expressed *in vivo* at both 1 month and 2 months after injection in mice. GFP staining in the cortex of P0-injected mice was readily detected. This data suggests, that the AAV vector may be used to successfully and efficiently express the constructs described herein *in vivo.*

### Example 4. Anti-tau antibodies comprising different Fc domains maintain binding to tau

To determine how and if Fc domain affects antibody binding of HJ8.5 to tau, HJ8.5 constructs were developed comprising IgG1, IgG2b and IgG2ab. **FIG. 11A** shows a schematic of how the various constructs were constructed. Expression of the various constructs was confirmed via Western blotto the FLAG tag present (**FIG. 11B**). **FIG. 11C** **is** a Western blot showing that the HJ8.5 antibody comprising IgG1, IgG2b or IgG2ab maintained its binding affinity for tau. Further, **FIG. 11D** shows that tau immunoprecipitated with each of the HJ8.5 antibody constructs comprising IgG1, IgG2b or IgG2ab. Accordingly, these results demonstrate that anti-tau antibody HJ8.5 comprising different Fc domains maintains its binding affinity for tau.

### Example 5. AAV constructs can be expressed throughout the brain

To examine the breadth of expression througout the brain, a vector for a construct comprising a secretory signal peptide (SP), the variable region light chain (V_{L}) of HJ8.5, a spacer, the variable region heavy chain (V_{H}) of HJ8.5, an SC3 spacer, and an HA-tag (HA) was generated (SC3 scFv HJ8.5 HA AAV2/8). This construct may be used to express the scFv in the brain for rapid assessment. **Fig. 12** depicts an anti-HA staining of brain sections from a mouse that was injected with SC3 scFv HJ8.5 HA AAV2/8 into the ventricles at post-natal day (P)0 and euthanized 3 months later. This data shows that the AAV vector may be used to successfully and efficiently express the scFvs constructs described herein throughout the brain.

### Example 6. scFv's of anti-tau antibody HJ8.5 are expressed and secreted in vivo

As discussed above, **FIG. 1** depicts exemplary scFv derivatives of HJ8.5. The scFv's comprise a secretory signal peptide (SP) for secretion of the protein, the variable region light chain (V_{L}) of HJ8.5, a spacer, the variable region heavy chain (V_{H}) of HJ8.5 and an HA-tag (HA). SC1 comprises the spacer (GGGS)₁; and SC3 comprises the spacer S(GGGS)₃. Additional spacers may be used.

To confirm that the scFv's described herein are both efficiently secreted in vivo, mice were injected with either SC1 scFv HJ8.5 HA AAV2/8 or SC3 scFv HJ8.5 HA AAV2/8 into the ventricles at P0. **Fig. 13** depicts a Western Blot of cortex lysates (top panel) from mice injected with the different vectors. All injected mice show expression of the scFvs. Immunoprecipitation experiments (bottom panel) with an HA antibody showed co-precipitation of human tau from mice that expressed the human P301S tau transgene, but not wild type mice (bottom panel).

### Example 7. Distribution of scFv expressed from constructs in the brain

To determine whether constructs expressed in the brain would have systemic distribution, plasma samples were taken from mice injected with the vectors. **Fig. 14** depicts a Western Blot of plasma samples of mice injected with SC1 scFv HJ8.5 HA AAV2/8 (top panel) or SC3 scFv HJ8.5 HA AAV2/8 (bottom panel) into the ventricles of the mice brains at P0. The Blot was stained with an HA antibody. At the left of the Blots are controls. These results show that scFvs are not detectable in the plasma and the vectors do not result in systemic distribution of the constructs from expression in the brain.

To determine whether constructs expressed in the brain would be present in cerebrospinal fluid (CSF) or in interstitial fluid (ISF), mice were injected with vectors of the scFv constructs as described above. CSF and ISF were subsequently collected from the mice. **Fig. 15** depicts a Western Blot of CSF samples (top panel) of mice injected with SC1 scFv HJ8.5 HA AAV2/8 (top panel), SC3 scFv HJ8.5 HA AAV2/8 or PBS into the ventricles at P0. In these samples, only SC3 scFv HJ8.5 HA could be detected. An immunoprecipitation experiment with a HA antibody (bottom panel) revealed the presence of SC3 scFv HJ8.5 HA but not SC1 scFv HJ8.5 HA in interstitial fluid (ISF) samples from these mice. This indicates that the construct with a longer linker was more readily distributed in CSF and ISF,

### Example 8. Polypeptides of scFv's of anti-tau antibody HJ8.5 with other functional domains

To determine whether other tau-binding polypeptides with other functional domains could be expressed *in vivo,* 293t cells were transfected with vectors for constructs comprising scFv's of anti-tau antibody HJ8.5, as well as a polynucleotide encoding tau. More particularly, constructs were developed comprising polynucleotides expressing SC1 scFv HJ8.5 HA, SC2 scFv HJ8.5 HA, and SC3 scFv scFv HJ8.5 HA fused to HSC-binding motifs for chaperone-mediated autophagy (hereinfafter; SC1 scFv-HSC, SC2 scFv-HSC, and SC3 scFv-HSC). Constructs were also developed expressing SC1 scFv HJ8.5 HA, SC2 scFv HJ8.5 HA, and SC3 scFv scFv HJ8.5 HA fused to either ubiquitin having a K48R point mutation for lysosomal degradation (hereinfafter, SC1 scFv-UB K48, SC2 scFv-UB K48, and SC3 scFv-UB K48), or ubiquitin having a K63R point mutation for proteasomal degradation (hereinafter, SC1 scFv-UB K63, SC2 scFv-UB K63, and SC3 scFv-UB K63). **Fig. 16** depicts a Western Blot of these scFv HJ8.5 constructs (top panel - A) Western blot from lysate of 293t cells transfected with tau and co-transfected with a vector for one of SC1 scFv HJ8.5 HA, SC2 scFv HJ8.5 HA, SC3 scFv scFv HJ8.5 HA, SC1 scFv-HSC, SC2 scFv-HSC, or SC3 scFv-HSCSG1. (bottom panel - B) Western blot from lysate of 293t cells transfected with tau and co-transfected with a vector for SC1 scFv-UB K48, SC2 scFv-UB K48, SC3 scFv-UB K48, SC1 scFv-UB K63, SC2 scFv-UB K63, or SC3 scFv-UB K63. As shown in Fig. 16, each of the constructs with different functional domains were successfully expressed *in vivo.*

### Example 9. Anti-tau antibodies comprising different Fc domains are expessed in vivo

To determine if vectors for antibodies with different Fc domains would be expressed *in vivo,* vectors for chimeric antibodies having the binding domain of HJ8.5 were transfected into CHOK1 cells. Four cHJ8.5 antibody constructs were developed, comprising IgG2ab, IgG2abD265A, IgG1, or IgG1D265A. Each construct was C-terrninally Flag tagged at the heavy and light IgG chains. **Fig. 17** depicts a Western blot showing that the cHJ8.5 antibody constructs are expressed and secreted upon transfection in CHOK1 cells,

**Fig. 18** depicts Western blots showing characterization of these AAV-cHJ8.5 IgG Fc variants in primary cultures. Panel A depicts expression of full-length chimeric cHJ8.5 IgG2ab, IgG2abD265A, IgG1, and IgG1D265A constructs following infection with respective AAV2/8-cHJ8.5 viruses in primary neurons and glia cultures. Panel B depicts the supernatant from the primary cultures can be used to detect human tau run on a Western blot in brain lysate from P301S human tau transgenic mice. Accordingly, these results demonstrate that anti-tau antibody HJ8.5 comprising different Fc domains are successfully expressed *in vivo.*

### Example 10. Expression and Secrection of anti-tau antibody comprising IgG2ab

**Fig. 19** depicts Western blots characterizing expression and secretion of AAV2/8-cHJ8.5 IgG2ab in vivo. Panel A depicts a Western blot of cortical brain lysate from a P301S human tau transgenic mice and their WTlittermate injected at postnatal day 0 (P0) with AAV-cHJ8.5 IgG2ab-Flag. Protein G effectively immunoprecipitates anti-tau cHJ8.5 heavy and light chains from P301S and control brain lysates. Panel B shows that protein G co-immunoprecipitates cHJ8.5 and human tau in P301S brain lysates. Panel C depicts cHJ8.5-Flag is secreted and detected in the plasma of mice injected with AAV2/8 cHJ8.5. Accordingly, these results demonstrate that an anti-tau antibody having the binding domain of HJ8.5 and comprising a different Fc domains is successfully expressed *in vivo* and binds ta u.

### Example 11. IHC Images Showing Expression and Secrection of Anti-tau Antibodies With Different Fc Regions

**Fig. 20** depicts immunohistochemistry images which demonstrate the wide-spread expression of AAV-cHJ8.5 IgG2ab-Flag (upper panel) versus AAV-control (lower panel) in treated P301S brain sections at 5 weeks post injection as indicated by probing for either anti-flag (green) or anti-IgG mouse (red). The merged IHC result is shown by yellow at right.

**Fig. 21** depicts immunohistochemistry images which demonstrate wide-spread expression of AAV-cHJ8.5 IgG1-Flag treated brain sections at 12 weeks post injection as indicated by probing for anti-flag,

**Fig, 22** depicts expression of all cHJ8.5 IgG Fc variants C termianlly Flag tagged in vivo following injection with AAV2/8-cHJ8.5-Flag viruses at P0. Panel A depicts immunohistochemistry images of AAV-cHJ8.5 IgG Fc variant treated mouse brain sections at 14 days post-injection as indicated by probing with anti-Flag. Panel B depicts expression of cHJ8.5 variants in mice 14 days post-injection by Western blot in cortex brain lysate (upper panel) and in plasma (lower panel).

**Fig. 23** depicts immunohistochemistry images which demonstrates wide-spread expression of AAV2/8-cHJ8.5 IgG2ab-Flag treated brain sections at 9 months post-injection as indicated by probing with anti-IgG mouse. (A) depicts expression in the hippocampus of 9 month old mice while (B) depicts expression in the entorhinal cortex of 9 month old mice expressing cHJ8.5 IgG2ab.

These results indicate that the vector for the anti-tau antibody continues to be expressed *in vivo* for a significant periods post-injection, including at least nine months after initial injection.

**Table 1. Sequence listings**

| | |
|---|---|
| SEQ ID NO: 1 | DRKDOGGYTMHQD |
| SEQ ID NO: 2 | KTDHGAE |
| SEQ ID NO: 3 | PRHLSNV |
| SEQ ID NO: 4 | EPRQ |
| SEQ ID NO: 5 | AAGHV |
| SEQ ID NO: 6 | TDHGAEIVYKSPVVSG |
| SEQ ID NO: 7 | EFEVMED |
| SEQ ID : NO: 8 | GGKVQHNKK |
| SEQ ID NO: 9 | SKIGSTENLKH |
| SEQ ID NO: 10 | TDHGAE |
| SEQ ID NO; 11 | KTDHGA |
| SEQ ID NO: 12 | GACATTGTGCTGACACAGTCTCCTGCTTCCTTAGCTGTATCT CTGGGACAGAGGGCCACCATCTCATGCAGGGCCAGCCAAAG TGTCAGTACATCTAGATATAGTTATATACACTGGTACCAACAG AAACCAGGACAGCCACCCAAACTCCTCATCAAGTATGCATCC AACCTAGAATCTGGGGTCCCTGCCAGGTTCAGTGGCAGTGG GTCTGGGACAGACTTCACCCTCAACATCCATCCTCTGGAGGA GGAGGATGCTGCAACATATTACTGTCACCACAGTTGGGAGAT TCCGCTCACGTTCGGTGCTGGGACCAAGCTGGAGCTGAAA 1. |
| SEQ ID NO: 13 | GAAGTGAAGGTTGAGGAGTCTGGAGGAGGCTTGGTGCAACCTGGAGG ATCCATGAAACTCTCCTGTGTTGTCTCTGGATTCACTTTCAGTAACTACT GGGTGAACTGGGTCCGCCAGTCTCCAGAGAAGGGGCTTGAGTGGGTT GCTCAAATTAGATTGAAATCTGATAATTATGCAACACATTATGAGGAGT CTGTGAAAGGGAGGTTCACCATCTCAAGAGATGATTCCAAAAGTAGTG TCTATCTGCAAATGAACAACCTAAGGGCTGAAGACAGTGGAATTTATTA CTGCACTAACTGGGAAGACTACTGGGGCCAAGGCACCACTCTCACAGT CTCCTCA |
| SEQ ID NO: 14 | Asp Ile Val Leu Thr Gln Ser Pro Ala Ser Leu Ala Val Ser Leu Gly Gln Arg Ala Thr Ile Ser Cys Arg Ala Ser Gln Ser Val Ser Thr Ser Arg Tyr Ser Tyr Ile His Trp Tyr Gln Gln Lys Pro Gly Gln Pro Pro Lys Leu Leu Ile Lys Tyr Ala Ser Asn Leu Glu Ser Gly Val Pro Ala Arg Phe Ser Gly Ser Gly Ser Gly Thr Asp Phe Thr Leu Asn Ile His Pro Leu Glu Glu Glu Asp Ala Ala Thr Tyr Tyr Cys His His Ser Trp Glu Ile Pro Leu Thr Phe Gly Ala Gly Thr Lys Leu Glu Leu Lys |
| SEQ ID NO: 15 | Glu Val Lys Val Glu Glu Ser Gly Gly Gly Leu Val Gln Pro Gly Gly Ser Met Lys Leu Ser Cys Val Val Ser Gly Phe Thr Phe Ser Asn Tyr Trp Val Asn Trp Val Arg Gln Ser Pro Glu Lys Gly Leu Glu Trp Val Ala Gln Ile Arg Leu Lys Ser Asp Asn Tyr Ala Thr His Tyr Glu Glu Ser Val Lys Gly Arg Phe Thr Ile Ser Arg Asp Asp Ser Lys Ser Ser Val Tyr Leu Gln Met Asn Asn Leu Arg Ala Glu Asp Ser Gly Ile Tyr Tyr Cys Thr Asn Trp Glu Asp Tyr Trp Gly Gln Gly Thr Thr Leu Thr Val Ser Ser |
| SEQ ID NO: 16 | Arg Ala Ser GIn Ser Val Ser Thr Ser Arg Tyr Ser Tyr Ile His |
| SEQ ID NO: 17 | Tyr Ala Ser Asn Leu Glu Ser |
| SEQ ID NO: 18 | His His Ser Trp Glu Ile Pro Leu Thr |
| SEQ ID NO: 19 | Asn Tyr Trp Val Asn |
| SEQ ID NO: 20 | Gln Ile Arg Leu Lys Ser Asp Asn Tyr Ala Thr His Tyr Glu Glu Ser Val Lys Gly |
| SEQ ID NO: 21 | Trp Glu Asp Tyr |
| SEQ ID NO: 21 | MDMRVPAQLLGLLLLWLRGARG |
| SEQ ID NO: 22 | ATGGATATGAGAGTGCCTGCCCAACTTCTCGGACTGCTGCTGCTTTGG CTTAGAGGTGCAAGATGC |
| SEQ ID NO: 23 | MLTPPLLLLLPLLSALVAAAIDAP |
| SEQ ID NO: 24 | ATGCTGACCCCGCCGTTGCTCCTGCTGCTGCCCCTGCTCTCAGCTCTG GTCGCGGCGGCTATC 2. |
| SEQ ID NO: 25 | TEELRVRLASHLRKLRKRLLRDA |
| SEQ ID NO: 26 | ACCGAGGAGCTGCGGGTGCGCCTCGCCTCCCACCTGCGCAAGC TGCGTAAGCGGCTCCTCCGCGATGCC |
| SEQ ID NO: 27 | SSVIDALQYKLEGTTRLTRKRGLKLATALSLSNKFVEGS |
| SEQ ID NO: 28 | TCATCTGTCATTGATGCACTGCAGTACAAATTAGAGGGCACCAC AAGATTGACAAGAAAAAGGGGATTGAAGTTAGCCACAGCTCTGT CTCTGAGCAACAAATTTGTGGAGGGTAGT |
| SEQ ID NO: 29 | SCATNASICGDEARCVRTEKAAYCACRSGFHTVPGQPGCQDINECLRFGT CSQLCNNTKGGHLCSCARNFMKTHNTCKAEGSEYQVLYIADDNEIRSLFP GHPHSAYEQAFQGDESVRIDAMDVHVKAGRVYWTNWHTGTISYRSLPPA APPTTSNRHRRQIDRGVTHLNISGLKMPRGIAIDWVAGNVYWTDSGRDVI EVAQMKGENRKTLISGMIDEPHAIVVDPLRGTMYWSDWGNHPKIETAAMD GTLRETLVQDNIQWPTGLAVDYHNERLYWADAKLSVIGSIRLNGTDPIVAA DSKRGLSHPFSIDVFEDYIYGVTYINNRVFKIHKFGHSPLVNLTGGLSHASD VVLYHQHKQPEVTNPCDRKKCEWLCLLSPSGPVCTCPNGKRLDNGTCVP VPSPTPPPDAPRPGTCNLQCFNGGSCFLNARRQPKCRCQPRYTGDKCEL DQCWEHCRNGGTCAASPSGMPTCRCPTGFTGPKCTQQVCAGYCANNS TCTVNQGNQPQCRCLPGFLGDRCQYRQCSGYCENFGTCQMAADGSRQ CRCTAYFEGSRCEVNKCSRCLEGACVVNKQSGDVTCNCTDGRVAPSCLT CVGHCSNGGSCTMNSKMMPECQCPPHMTGPRCEEHVFSQQQPGHIASI LIPLLLLLLLVLVAGVVFWYKRRVQGAKGFQHQRMTNGAMNVEIGNPTYK MYEGGEPDDVGGLLDADFALDPDKPTNFTNPVYATLYMGGHGSRHSLAS TDEKRELLGRGPEDEIGDPLAYPYDVPDYA 3. |
| SEQ ID NO: 30 | agctgcgcgaccaacgcgagcatttgcggcgatgaagcgcgctgcgtgcgcaccgaaaaagcggcg tattgcgcgtgccgcagcggctttcataccgtgccgggccagccgggctgccaggatattaacgaatgc ctgcgctttggcacctgcagccagctgtgcaacaacaccaaaggcggccatctgtgcagctgcgcgcg caactttatgaaaacccataacacctgcaaagcggaaggcagcgaatatcaggtgctgtatattgcgga tgataacgaaattcgcagcctgtttccgggccatccgcatagcgcgtatgaacaggcgtttcagggcgat gaaagcgtgcgcattgatgcgatggatgtgcatgtgaaagcgggccgcgtgtattggaccaactggcat |
| | accggcaccattagctatcgcagcctgccgccggcggcgccgccgaccaccagcaaccgccatcgc cgccagattgatcgcggcgtgacccatctgaacattagcggcctgaaaatgccgcgcggcattgcgatt gattgggtggcgggcaacgtgtattggaccgatagcggccgcgatgtgattgaagtggcgcagatgaa aggcgaaaaccgcaaaaccctgattagcggcatgattgatgaaccgcatgcgattgtggtggatccgct gcgcggcaccatgtattggagcgattggggcaaccatccgaaaattgaaaccgcggcgatggatggc accctgcgcgaaaccctggtgcaggataacattcagtggccgaccggcctggcggtggattatcataac gaacgcctgtattgggcggatgcgaaactgagcgtgattggcagcattcgcctgaacggcaccgatccg attgtggcggcggatagcaaacgcggcctgagccatccgtttagcattgatgtgtttgaagattatatttatg gcgtgacctatattaacaaccgcgtgtttaaaattcataaatttggccatagcccgctggtgaacctgaccg gcggcctgagccatgcgagcgatgtggtgctgtatcatcagcataaacagccggaagtgaccaacccg tgcgatcgcaaaaaatgcgaatggctgtgcctgctgagcccgagcggcccggtgtgcacctgcccgaa cggcaaacgcctggataacggcacctgcgtgccggtgccgagcccgaccccgccgccggatgcgcc gcgcccgggcacctgcaacctgcagtgctttaacggcggcagctgctttctgaacgcgcgccgccagc cgaaatgccgctgccagccgcgctataccggcgataaatgcgaactggatcagtgctgggaacattgc cgcaacggcggcacctgcgcggcgagcccgagcggcatgccgacctgccgctgcccgaccggcttta ccggcccgaaatgcacccagcaggtgtgcgcgggctattgcgcgaacaacagcacctgcaccgtgaa ccagggcaaccagccgcagtgccgctgcctgccgggctttctgggcgatcgctgccagtatcgccagtg cagcggctattgcgaaaactttggcacctgccagatggcggcggatggcagccgccagtgccgctgca ccgcgtattttgaaggcagccgctgcgaagtgaacaaatgcagccgctgcctggaaggcgcgtgcgtg gtgaacaaacagagcggcgatgtgacctgcaactgcaccgatggccgcgtggcgccgagctgcctga cctgcgtgggccattgcagcaacggcggcagctgcaccatgaacagcaaaatgatgccggaatgcca gtgcccgccgcatatgaccggcccgcgctgcgaagaacatgtgtttagccagcagcagccgggccata ttgcgagcattctgattccgctgctgctgctgctgctgctggtgctggtggcgggcgtggtgttttggtataaa cgccgcgtgcagggcgcgaaaggctttcagcatcagcgcatgaccaacggcgcgatgaacgtggaa attggcaacccgacctataaaatgtatgaaggcggcgaaccggatgatgtgggcggcctgctggatgc ggattttgcgctggatccggataaaccgaccaactttaccaacccggtgtatgcgaccctgtatatgggcg gccatggcagccgccatagcctggcgagcaccgatgaaaaacgcgaactgctgggccgcggcccgg aagatgaaattggcgatccgctggcgtatccgtatgatgtgccggattatgcg |
| SEQ ID NO: 31 | YPYDVPDYA |
| SEQ ID NO: 32 | DQGGYT |

Further aspects of the invention:
1. A polynucleotide sequence encoding a polypeptide, the polypeptide comprising at least one tau binding moiety attached to a targeting moiety via a linker and optionally comprising a signal peptide and/or a purification moiety, wherein:
   (a) each tau binding moiety is independently selected from the group consisting of a VH fragment, a VL fragment, a Fv fragment, a single-chain variable fragment (scFv), a minibody, a diabody, a triabody, and a tetrabody;
   (b) the targeting moiety is selected from the group consisting of:
      (i) a polypeptide that binds to the low-density lipoprotein receptor (LDLR),
      (ii) a polypeptide that binds to the low-density lipoprotein receptor-related protein (LRP1),
      (iii) a polypeptide comprising the transmembrane and intracellular domain of LRP1 or LDLR;
      (iv) a polypeptide comprising a HSC-binding motif, and
      (v) ubiquitin or ubiquitin mutant;
   (c) the linker has the polypeptide sequence (GGGS/T)ₙ or S/T(GGGS/T)ₙ, wherein *n* is an integer from 1 to 6, inclusive.
2. The polynucleotide sequence of aspect 1, wherein the ubiquitin mutant comprises a K48R point mutation or a K63R point mutation.
3. The polynucleotide sequence of aspect 1, wherein the targeting moiety is selected from the group consisting of:
   (i) a polypeptide that binds to the low-density lipoprotein receptor (LDLR),
   (ii) a polypeptide that binds to the low-density lipoprotein receptor-related protein (LRP1), and
   (iii) a polypeptide comprising the transmembrane and intracellular domain of LRP1 or LDLR.
4. The polynucleotide sequence of any of the preceding aspects, wherein at least one tau binding moiety of the polypeptide specifically binds an antigenic determinant of tau selected within an amino acid selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, and SEQ ID NO: 8.
5. The polynucleotide sequence of any of the preceding aspects, wherein at least one tau binding moiety of the polypeptide comprises a V_{L} fragment and the V_{L} fragment comprises a light chain CDR1 comprising the amino acid sequence of SEQ ID NO: 16 with zero to two amino acid substitutions, a light chain CDR2 comprising the amino acid sequence of SEQ ID NO: 17 with zero to two amino acid substitutions, a light chain CDR3 comprising the amino acid sequence of SEQ ID NO: 18 with zero to two amino acid substitutions, or any combination thereof.
6. The polynucleotide sequence of any of the preceding aspects, wherein at least one tau binding moiety of the polypeptide comprises a V_{H} fragment and the V_{H} fragment comprises a heavy chain CDR1 comprising the amino acid sequence of SEQ ID NO: 19 with zero to two amino acid substitutions, a heavy chain CDR2 comprising the amino acid sequence of SEQ ID NO: 20 with zero to two amino acid substitutions, a heavy chain CDR3 comprising the amino acid sequence of SEQ ID NO: 21 with zero to two amino acid substitutions, or any combination thereof.
7. The polynucleotide sequence of any of the preceding aspects, wherein at least one tau binding moiety of the polypeptide has at least 80%, at least 85%, at least 90%, at least 95%, at least 98%, at least 99% or more sequence identity to SEQ ID NO: 14.
8. The polynucleotide sequence of any of the preceding aspects, wherein at least one tau binding moiety of the polypeptide has at least 80%, at least 85%, at least 90%, at least 95%, at least 98%, at least 99% or more sequence identity to SEQ ID NO: 15.
9. The polynucleotide sequence any of the preceding aspects, wherein at least one tau binding moiety of the polypeptide is a scFv and the scFv comprises SEQ ID NO: 14 and SEQ ID NO: 15.
10. The polynucleotide sequence of aspect 9, wherein the scFv comprises SEQ ID NO: 14 attached to SEQ ID NO: 15 via a polypeptide sequence, wherein the polypeptide sequence is (GGGS/T)ₙ or S/T(GGGS/T)ₙ, and *n* is an integer from 1 to 6, inclusive.
11. The polynucleotide sequence of any of the preceding aspects, wherein the targeting moiety comprises an amino acid sequence that has at least 80%, at least 85%, at least 90%, or at least 95% sequence identity to SEQ ID NO: 27 (SSVIDALQYKLEGTTRLTRKRGLKLATALSLSNKFVEGS).
12. The polynucleotide sequence of any of the preceding aspects, wherein the targeting moiety comprises an amino acid sequence that has at least 80%, at least 85%, at least 90%, or at least 95% sequence identity to SEQ ID NO: 25 (TEELRVRLASHLRKLRKRLLRDA).
13. The polynucleotide sequence of any of the preceding aspects, wherein the targeting moiety comprises the transmembrane and intracellular domains of LRP1 or LDLR.
14. The polynucleotide sequence of any of the preceding aspects, wherein the targeting moiety comprises an amino acid sequence that has at least 80%, at least 85%, at least 90%, or at least 95% sequence identity to SEQ ID NO: 29.
15. The polynucleotide sequence of aspect 1, wherein:
   (a) at least one tau binding moiety is an scFv comprising SEQ ID NO: 14 and SEQ ID NO: 15; the targeting moiety has at least 80% sequence identity to SEQ ID NO: 25; and the linker is the amino acid sequence S/T(GGGS/T)ₙ, wherein *n* is an integer from 1 to 6, inclusive;
   (b) at least one tau binding moiety is an scFv comprising SEQ ID NO: 14 and SEQ ID NO: 15; the targeting moiety has at least 80% sequence identity to SEQ ID NO: 27, and the linker is the amino acid sequence S/T(GGGS/T)ₙ, wherein *n* is an integer from 1 to 6, inclusive;
   (c) at least one tau binding moiety is an scFv comprising SEQ ID NO: 14 and SEQ ID NO: 15; the targeting moiety comprises the transmembrane and intracellular domain of LRP1 or LDLR; and the linker is the amino acid sequence S/T(GGGS/T)ₙ, wherein *n* is an integer from 1 to 6, inclusive;
   (d) at least one tau binding moiety is an scFv comprising SEQ ID NO: 14 attached to SEQ ID NO: 15 via a polypeptide sequence, wherein the polypeptide sequence is (GGGS/T)ₙ or S/T(GGGS/T)ₙ, and *n* is an integer from 1 to 6, inclusive; the targeting moiety has at least 80% sequence identity to the apoB-BD sequence; and the linker is the amino acid sequence (GGGS/T)ₙ, wherein *n* is an integer from 1 to 6, inclusive;
   (e) at least one tau binding moiety is an scFv comprising SEQ ID NO: 14 attached to SEQ ID NO: 15 via a polypeptide sequence, wherein the polypeptide sequence is (GGGS/T)ₙ or S/T(GGGS/T)ₙ, and *n* is an integer from 1 to 6, inclusive; the targeting moiety has at least 80% sequence identity to the ApoE-BD sequence; and the linker is the amino acid sequence (GGGS/T)ₙ, wherein *n* is an integer from 1 to 6, inclusive; or
   (f) at least one tau binding moiety is an scFv comprising SEQ ID NO: 14 attached to SEQ ID NO: 15 via a polypeptide sequence, wherein the polypeptide sequence is (GGGS/T)ₙ or S/T(GGGS/T)ₙ, and *n* is an integer from 1 to 6, inclusive; the targeting moiety comprises the transmembrane and intracellular domains of LRP1 or LDLR; and the linker is the amino acid sequence (GGGS/T)ₙ, wherein *n* is an integer from 1 to 6, inclusive.
16. A polynucleotide sequence encoding a polypeptide of any of the preceding aspects, wherein the polypeptide comprises a signal peptide at the N-terminus.
17. A polynucleotide sequence encoding a polypeptide of any of the preceding aspects, wherein the polypeptide comprises a purification moiety at the C-terminus.
18. An isolated polypeptide sequence encoded by a polynucleotide sequence of any of the preceding aspects.
19. A vector comprising a polynucleotide sequence of any of the preceding aspects.
20. The vector of aspect 19, wherein the vector is an AAV vector.
21. An AAV vector, wherein the AAV vector encodes a polypeptide, the polypeptide comprising at least one tau binding moiety and optionally comprising a signal peptide and/or a purification moiety, wherein each tau binding moiety is independently selected from the group consisting of a VH fragment, a VL fragment, a Fv fragment, a single-chain variable fragment (scFv), a minibody, a diabody, a triabody, and a tetrabody.
22. The AAV vector of aspect 21, wherein the vector further comprises a targeting moiety and a linker, such that the tau binding moiety and the targeting moiety are attached via the linker, wherein:
   (a) the targeting moiety is selected from the group consisting of:
      (i) a polypeptide that binds to the low-density lipoprotein receptor (LDLR),
      (ii) a polypeptide that binds to the low-density lipoprotein receptor-related protein (LRP1), and
      (iii) a polypeptide comprising the transmembrane and intracellular domain of LRP1 or LDLR;
      (iv) a polypeptide comprising a HSC-binding motif, and
      (v) ubiquitin or ubiquitin mutant;
   (b) the linker has the polypeptide sequence (GGGS/T)ₙ or S/T(GGGS/T)ₙ, wherein *n* is an integer from 1 to 6, inclusive.
23. The AAV vector of aspect 22, wherein the ubiquitin mutant comprises a K48R point mutation or a K63R point mutation.
24. The AAV vector of aspect 21, wherein the vector further comprises a targeting moiety and a linker, such that the tau binding moiety and the targeting moiety are attached via the linker, wherein:
   the targeting moiety is selected from the group consisting of:
   (i) a polypeptide that binds to the low-density lipoprotein receptor (LDLR),
   (ii) a polypeptide that binds to the low-density lipoprotein receptor-related protein (LRP1), and
   (iii) a polypeptide comprising the transmembrane and intracellular domain of LRP1 or LDLR.
25. The AAV vector of any of aspects 21 to 24, wherein at least one tau binding moiety of the polypeptide specifically binds an antigenic determinant of tau selected within an amino acid selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, and SEQ ID NO: 8.
26. The AAV vector of any of aspects 21 to 25, wherein at least one tau binding moiety of the polypeptide comprises a V_{L} fragment and the V_{L} fragment comprises a light chain CDR1 comprising the amino acid sequence of SEQ ID NO: 16 with zero to two amino acid substitutions, a light chain CDR2 comprising the amino acid sequence of SEQ ID NO: 17 with zero to two amino acid substitutions, a light chain CDR3 comprising the amino acid sequence of SEQ ID NO: 18 with zero to two amino acid substitutions, or any combination thereof.
27. The AAV vector of any of aspects 21 to 26, wherein at least one tau binding moiety of the polypeptide comprises a V_{H} fragment and the V_{H} fragment comprises a heavy chain CDR1 comprising the amino acid sequence of SEQ ID NO: 19 with zero to two amino acid substitutions, a heavy chain CDR2 comprising the amino acid sequence of SEQ ID NO: 20 with zero to two amino acid substitutions, a heavy chain CDR3 comprising the amino acid sequence of SEQ ID NO: 21 with zero to two amino acid substitutions, or any combination thereof.
28. The AAV vector of any of aspects 21 to 27, wherein at least one tau binding moiety of the polypeptide has at least 80%, at least 85%, at least 90%, at least 95%, at least 98%, at least 99% or more sequence identity to SEQ ID NO: 14.
29. The AAV vector of any of aspects 21 to 28, wherein at least one tau binding moiety of the polypeptide has at least 80%, at least 85%, at least 90%, at least 95%, at least 98%, at least 99% or more sequence identity to SEQ ID NO: 15.
30. The AAV vector of any of aspects 21 to 29, wherein at least one tau binding moiety of the polypeptide is a scFv and the scFv comprises SEQ ID NO: 14 and SEQ ID NO: 15.
31. The AAV vector of any of aspects 21 to 30, wherein the scFv comprises SEQ ID NO: 14 attached to SEQ ID NO: 15 via a polypeptide sequence, wherein the polypeptide sequence is (GGGS/T)ₙ or S/T(GGGS/T)ₙ, and *n* is an integer from 1 to 6, inclusive.
32. The AAV vector of any of aspects 21 to 31, wherein the targeting moiety comprises an amino acid sequence that has at least 80%, at least 85%, at least 90%, or at least 95% sequence identity to SEQ ID NO: 27 (SSVIDALQYKLEGTTRLTRKRGLKLATALSLSNKFVEGS).
33. The AAV vector of any of aspects 21 to 32, wherein the targeting moiety comprises an amino acid sequence that has at least 80%, at least 85%, at least 90%, or at least 95% sequence identity to SEQ ID NO: 25 (TEELRVRLASHLRKLRKRLLRDA).
34. The AAV vector of any of aspects 21 to 33, wherein the targeting moiety comprises the transmembrane and intracellular domains of LRP1 or LDLR.
35. The AAV vector of any of aspects 21 to 34, wherein the targeting moiety comprises an amino acid sequence that has at least 80%, at least 85%, at least 90%, or at least 95% sequence identity to SEQ ID NO: 29.
36. The AAV vector of any of aspect 21, wherein:
   (a) at least one tau binding moiety is an scFv comprising SEQ ID NO: 14 and SEQ ID NO: 15; the targeting moiety has at least 80% sequence identity to SEQ ID NO: 25; and the linker is the amino acid sequence S/T(GGGS/T)ₙ, wherein *n* is an integer from 1 to 6, inclusive;
   (b) at least one tau binding moiety is an scFv comprising SEQ ID NO: 14 and SEQ ID NO: 15; the targeting moiety has at least 80% sequence identity to SEQ ID NO: 27, and the linker is the amino acid sequence S/T(GGGS/T)ₙ, wherein *n* is an integer from 1 to 6, inclusive;
   (c) at least one tau binding moiety is an scFv comprising SEQ ID NO: 14 and SEQ ID NO: 15; the targeting moiety comprises the transmembrane and intracellular domain of LRP1 or LDLR; and the linker is the amino acid sequence S/T(GGGS/T)ₙ, wherein *n* is an integer from 1 to 6, inclusive;
   (d) at least one tau binding moiety is an scFv comprising SEQ ID NO: 14 attached to SEQ ID NO: 15 via a polypeptide sequence, wherein the polypeptide sequence is (GGGS/T)ₙ or S/T(GGGS/T)ₙ, and *n* is an integer from 1 to 6, inclusive; the targeting moiety has at least 80% sequence identity to the apoB-BD sequence; and the linker is the amino acid sequence (GGGS/T)ₙ, wherein *n* is an integer from 1 to 6, inclusive;
   (e) at least one tau binding moiety is an scFv comprising SEQ ID NO: 14 attached to SEQ ID NO: 15 via a polypeptide sequence, wherein the polypeptide sequence is (GGGS/T)ₙ or S/T(GGGS/T)ₙ, and *n* is an integer from 1 to 6, inclusive; the targeting moiety has at least 80% sequence identity to the ApoE-BD sequence; and the linker is the amino acid sequence (GGGS/T)ₙ, wherein *n* is an integer from 1 to 6, inclusive; or
   (f) at least one tau binding moiety is an scFv comprising SEQ ID NO: 14 attached to SEQ ID NO: 15 via a polypeptide sequence, wherein the polypeptide sequence is (GGGS/T)ₙ or S/T(GGGS/T)ₙ, and *n* is an integer from 1 to 6, inclusive; the targeting moiety comprises the transmembrane and intracellular domains of LRP1 or LDLR; and the linker is the amino acid sequence (GGGS/T)ₙ, wherein *n* is an integer from 1 to 6, inclusive.
37. The AAV vector of any of aspects 21 to 36, wherein the polypeptide comprises a signal peptide at the N-terminus.
38. The AAV vector of any of aspects 21 to 37, wherein the polypeptide comprises a purification moiety at the C-terminus.
39. A recombinant AAV, wherein the rAAV is produced by a vector of any of aspects 21 to 38.
40. An isolated cell comprising a polynucleotide sequence of any of aspects 1 to 18.
41. An isolated cell comprising the vector of any of aspects 19 to 39.
42. A recombinant AAV (rAAV), wherein the rAAV comprises a polynucleotide sequence of any of aspects 1 to 18.
43. A method of delivering to a cell a polynucleotide encoding a polypeptide comprising at least one tau binding moiety, the method comprising contacting the cell with a composition comprising a vector of any of aspects 19 to 39.
44. A method of delivering a tau binding moiety to an intracellular vesicle in a subject, the method comprising administering to the subject a composition comprising a polynucleotide of any of aspects 1 to 18.
45. A method of delivering a tau binding moiety to an intracellular vesicle in a subject, the method comprising administering to the subject a composition comprising a vector of any of aspects 19 to 39.
46. A method of delivering a tau binding moiety to an intracellular vesicle in a subject, the method comprising administering to the subject a composition comprising a rAAV of aspect 39 or aspect 42.
47. A polynucleotide sequence encoding an anti-tau antibody, the antibody comprising a variable region and a constant region, wherein
   the variable region specifically binds an antigenic determinant of tau selected within an amino acid selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, and SEQ ID NO: 8, and
   the constant region is an IgG1, IgG2ab, IgG2abD265A, or IgG2b constant region.
48. The polynucleotide of aspect 47, wherein the variable reign comprises a V_{L} fragment and the V_{L} fragment comprises a light chain CDR1 comprising the amino acid sequence of SEQ ID NO: 16 with zero to two amino acid substitutions, a light chain CDR2 comprising the amino acid sequence of SEQ ID NO: 17 with zero to two amino acid substitutions, a light chain CDR3 comprising the amino acid sequence of SEQ ID NO: 18 with zero to two amino acid substitutions, or any combination thereof.
49. The polynucleotide of any of aspects 47 to 48, wherein the variable region comprises a V_{H} fragment and the V_{H} fragment comprises a heavy chain CDR1 comprising the amino acid sequence of SEQ ID NO: 19 with zero to two amino acid substitutions, a heavy chain CDR2 comprising the amino acid sequence of SEQ ID NO: 20 with zero to two amino acid substitutions, a heavy chain CDR3 comprising the amino acid sequence of SEQ ID NO: 21 with zero to two amino acid substitutions, or any combination thereof.
50. A method of delivering an anti-tau antibody to an intracellular vesicle in a subject, the method comprising administering to the subject a composition comprising a composition or vector comprising the polynucleotide of any of aspects 47 to 49.
51. The method of aspect 50, wherein the vector is an AAV vector.

## Claims

1. A polynucleotide sequence encoding a polypeptide, the polypeptide comprising at least one tau binding moiety attached to a targeting moiety via a linker and optionally comprising a signal peptide and/or a purification moiety, wherein:
(a) the at least one tau binding moiety is independently selected from the group consisting of a Fab fragment, a F(ab')₂ fragment, a VH fragment, a VL fragment, a Fv fragment, a single-chain variable fragment (scFv), a minibody, a diabody, a triabody, and a tetrabody;
(b) the targeting moiety is selected from the group consisting of:
(i) a polypeptide that binds to the low-density lipoprotein receptor (LDLR),
(ii) a polypeptide that binds to the low-density lipoprotein receptor-related protein (LRP1),
(iii) a polypeptide comprising the transmembrane and intracellular domain of LRP1 or LDLR;
(iv) a polypeptide comprising a Heat Shock Cognate protein (HSC)-binding motif, and
(v) ubiquitin or a ubiquitin mutant;
(c) the linker has the polypeptide sequence (GGGS/T)ₙ (SEQ ID NO: 40) or S/T(GGGS/T)ₙ (SEQ ID NO: 41), wherein *n* is an integer from 1 to 6, inclusive.

2. The polynucleotide sequence of claim 1, wherein:
(a) the targeting moiety is a ubiquitin mutant which comprises a K48R point mutation or a K63R point mutation;
(b) the at least one tau binding moiety of the polypeptide specifically binds an antigenic determinant of tau selected within an amino acid selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, and SEQ ID NO: 8;
(c) the at least one tau binding moiety of the polypeptide comprises a V_{L} fragment and the V_{L} fragment comprises a light chain CDR1 comprising the amino acid sequence of SEQ ID NO: 16 with zero to two amino acid substitutions, a light chain CDR2 comprising the amino acid sequence of SEQ ID NO: 17 with zero to two amino acid substitutions, a light chain CDR3 comprising the amino acid sequence of SEQ ID NO: 18 with zero to two amino acid substitutions, or any combination thereof;
(d) the at least one tau binding moiety of the polypeptide comprises a V_{H} fragment and the V_{H} fragment comprises a heavy chain CDR1 comprising the amino acid sequence of SEQ ID NO: 19 with zero to two amino acid substitutions, a heavy chain CDR2 comprising the amino acid sequence of SEQ ID NO: 20 with zero to two amino acid substitutions, a heavy chain CDR3 comprising the amino acid sequence of SEQ ID NO: 21 with zero to two amino acid substitutions, or any combination thereof;
(e) the at least one tau binding moiety of the polypeptide has at least 80%, at least 85%, at least 90%, at least 95%, at least 98%, at least 99% or more sequence identity to SEQ ID NO: 14;
(f) the at least one tau binding moiety of the polypeptide has at least 80%, at least 85%, at least 90%, at least 95%, at least 98%, at least 99% or more sequence identity to SEQ ID NO: 15; and/or
(g) the at least one tau binding moiety of the polypeptide is a scFv and the scFv comprises a light chain variable region having the amino sequence of SEQ ID NO: 14 and a heavy chain variable region having the amino sequence of SEQ ID NO: 15,
optionally wherein the scFv comprises SEQ ID NO: 14 attached to SEQ ID NO: 15 via a spacer sequence, wherein the spacer sequence is (GGGS/T)ₙ (SEQ ID NO: 40) or S/T(GGGS/T)ₙ (SEQ ID NO: 41), and *n* is an integer from 1 to 6, inclusive.

3. The polynucleotide sequence of claim 1 or 2, wherein the targeting moiety comprises:
(a) an amino acid sequence that has at least 80%, at least 85%, at least 90%, or at least 95% sequence identity to SEQ ID NO: 27 (SSVIDALQYKLEGTTRLTRKRGLKLATALSLSNKFVEGS);
(b) an amino acid sequence that has at least 80%, at least 85%, at least 90%, or at least 95% sequence identity to SEQ ID NO: 25
(TEELRVRLASHLRKLRKRLLRDA);
(c) the transmembrane and intracellular domains of LRP1 or LDLR; and/or
(d) an amino acid sequence that has at least 80%, at least 85%, at least 90%, or at least 95% sequence identity to SEQ ID NO: 29.

4. A polynucleotide sequence encoding a polypeptide of any of the preceding claims, wherein the polypeptide comprises a signal peptide at the N-terminus and/or a purification moiety at the C-terminus.

5. An isolated polypeptide sequence encoded by a polynucleotide sequence of any of the preceding claims.

6. A vector comprising a polynucleotide sequence of any of the preceding claims, optionally wherein the vector is an AAV vector.

7. An adeno-associated virus (AAV) vector, wherein the AAV vector encodes a polypeptide, the polypeptide comprising at least one tau binding moiety and optionally comprising a signal peptide and/or a purification moiety, wherein the at least one tau binding moiety is independently selected from the group consisting of a Fab fragment, a F(ab')₂ fragment, a VH fragment, a VL fragment, a Fv fragment, a single-chain variable fragment (scFv), a minibody, a diabody, a triabody, and a tetrabody.

8. A recombinant AAV (rAAV), wherein:
(a) the rAAV is produced by the vector of claim 7; or
(b) the rAAV comprises a polynucleotide sequence of any of claims 1 to 4.

9. An isolated cell comprising a polynucleotide sequence of any of claims 1 to 4 or the vector of claim 6 or 7.

10. An *in vitro* method of delivering to an isolated cell a polynucleotide encoding a polypeptide comprising at least one tau binding moiety, the method comprising contacting the cell with a composition comprising the vector of claim 6 or 7.

11. The polynucleotide of any of claims 1 to 4 for use in a method of delivering a tau binding moiety to an intracellular vesicle in a subject, the method comprising administering to the subject a composition comprising the polynucleotide.

12. The vector of claim 6 or 7 for use in a method of delivering a tau binding moiety to an intracellular vesicle in a subject, the method comprising administering to the subject a composition comprising the vector.

13. The rAAV of claim 8 for use in a method of delivering a tau binding moiety to an intracellular vesicle in a subject, the method comprising administering to the subject a composition comprising the rAAV.

14. A polynucleotide sequence encoding an anti-tau antibody, the antibody comprising a variable region and a constant region, wherein
the variable region specifically binds an antigenic determinant of tau selected within an amino acid selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, and SEQ ID NO: 8, and
the constant region is an IgG1, IgG2ab, IgG2abD265A, or IgG2b constant region,
optionally wherein the variable region comprises:
(a) a V_{L} fragment and the V_{L} fragment comprises a light chain CDR1 comprising the amino acid sequence of SEQ ID NO: 16 with zero to two amino acid substitutions, a light chain CDR2 comprising the amino acid sequence of SEQ ID NO: 17 with zero to two amino acid substitutions, a light chain CDR3 comprising the amino acid sequence of SEQ ID NO: 18 with zero to two amino acid substitutions, or any combination thereof; and/or
(b) a V_{H} fragment and the V_{H} fragment comprises a heavy chain CDR1 comprising the amino acid sequence of SEQ ID NO: 19 with zero to two amino acid substitutions, a heavy chain CDR2 comprising the amino acid sequence of SEQ ID NO: 20 with zero to two amino acid substitutions, a heavy chain CDR3 comprising the amino acid sequence of SEQ ID NO: 21 with zero to two amino acid substitutions, or any combination thereof.

15. The polynucleotide of claim 14 for use in a method of delivering an anti-tau antibody to an intracellular vesicle in a subject, the method comprising administering to the subject a composition comprising a composition or vector comprising the polynucleotide, optionally wherein the vector is an AAV vector.
